# EUROPEAN PATENT APPLICATION

(11) **EP 1 607 477 A1**
(43) Date of publication of application: **21.12.2005**
(21) Application number: 05012911.3
(22) Date of filing: 15.06.2005
(51) Int. Cl.: C12N 9/00, C12N 15/52, G01N 33/573, A01N 61/00

(54) **Polynucleotides encoding insect acetyl coenzyme-A carboxylase and uses thereof**

(30) Priority: 15.06.2004 US 580038 P
(71) Applicant: Genoptera, LLC, South San Francisco, CA 94083-0511 (US); Bayer Cropscience LLP, Research Triangle Park, N.C. 27709 (US)
(72) Inventor: Franken, Eva-Maria, 42799 Leichlingen (DE); Weddell, Gregory, Vallejo, California 94591 (US); Johnston, Stuart, Menlo Park, California 94025 (US); Zhou, Lijuan, San Francisco, California 94122 (US); Denk, Dago Dimster, San Anselmo, California 94960 (US); Ebens, Allen J., Jr., San Francisco, California 94122 (US)
(74) Representative: Grünecker, Kinkeldey, Stockmair & Schwanhäusser Anwaltssozietät

(57) **Abstract**

The instant invention provides nucleic acid molecules encoding insect acetyl CoA carboxylase, as well as acetyl CoA carboxylase encoded thereby. The invention further provides methods of identifying agents that modulate a level of acetyl CoA carboxylase mRNA, polypeptide, or enzyme activity. Such agents are candidate insecticidal compounds.

SEQUENCE LISTING

<110> Franken, Eva-Maria, et al.

<120> Polynucleotides encoding insect
  acetyl-coenzyme-A carboxylase and uses thereof

<130> GNOP-012EP

<150> 60/580,038
<151> 2004-06-15

<160> 2

<170> FastSEQ for Windows Version 4.0

<210> 1
<211> 6876
<212> DNA
<213> Heliothis virescens

<400> 1
tcgaatgaaa actcatagga cattttacga gttgggacta tctgggccgt ctatgtccca 60
gggtacagtc atccatagcc agcggttcca agagaaagac ttcacggttg ctactcctga 120
ggagtttgtg aggcggttcc agggtactaa gcctattaat aaggtgttga tcgctaacaa 180
cggtattggt gcagtaaaat gcatgagatc tatcagaaga tggtcctacg agatgttcaa 240
gaacgaacgg gctgtcagat ttgttgttat ggtgactccc gaggatctga aggctaacgc 300
tgagtatatc aagatggccg accactatgt gcccgtgcct gggggctcta ataataataa 360
ctatgccaat gtcgagctga tcgtcgatat tgctattagg actcaagttc aggccgtatg 420
ggcaggatgg ggtcacgcat ccgagaaccc caagctcccc gaactgctgc accgtgctgg 480
ggtcgtgttc atcgggcccc ctgagaaagc catgtgggcc ttaggagaca agatcgcgtc 540
ctccatcgtc gctcaaactg ctgaaatacc tactttacct tggagtggca gtgaattgaa 600
agcggagtac aatagtaaga agataaaaat atcttctgaa ctcttcgcga agggttgtgt 660
caccaccccc gaacagggtc tacaagctgc ccagaagatt ggattcccgg tgatgatcaa 720
ggcttcagaa ggtggtggtg gcaagggtat caggaaagtc gacaatcctg atgatttcaa 780
cagcatgttc agacaggtac aagctgaagt tcccgggtct ccgatattcg tgatgaagct 840
ggcgaagtca gctcgccatt tggaagtgca actcctggct gaccaatatg gtaacgcgat 900
ctccctattc ggtcgtgact gttcgatcca gcgtcgtcat cagaagatca ttgaggaggc 960
tcccgcggcc atcgccaagc ctgatgtctt tattgagatg gaaaaggccg cagtccgtct 1020
agctaaaatg gtaggctacg tgtcagcagg cacagtggaa tacctgtacg agcccgcgac 1080
aggtgcttac tacttcctgg agctgaaccc tcggctgcag gtggaacatc cttgcactga 1140
gatggtggct gacgtcaact tgcccgctgc gcagttgcag atcgccatgg gtcttcccct 1200
ctaccacatt aaggatatcc gtctcctata cggagagtct ccatggggac tctctcaaat 1260
agagttcgat gagcccaagc aacgtccatc accctgggga cacgtcatcg cagccaggat 1320
cacttccgag aaccctgatg aaggattcaa gccatcatca ggaacagtcc aagagctgaa 1380
cttcaggtct tccaagaacg tgtggggcta cttcagcgtg gcagcatcag gaggtctcca 1440
cgagttcgcc gactcacagt tcggtcactg cttctcttgg ggcgagacca gggaacaggc 1500
cagagaaaac ttggtgatag ctctcaaaga gcttagcatc cgaggtgact tccgtaccac 1560
tgtggagtat ctgatcacgc tgctggagac tggagccttc cagaacaatg acatcgacac 1620
cgcctggctg gacgcgctta ttgctgagag gatgcaatca gagaagccgg atatcatgct 1680
gggtgtgatc tgcgggtcca ttctcatcgc cgacgcgtat atcaccgcca acttccagga 1740
gttcaagagt gcacttgaga agggtcaaat ccaaggttcc agcgccttat ccaactgcgt 1800
ggaagtggag ctgatccact cagggtccaa gtataaggtg tcggctacca agtccggccc 1860
gacctcctac ttcctcgcca tgaacggcag cttcaaggag atggaggtgc acaagcttac 1920
tgatggcggc atgctgctct caatcgacgg agcatcctac accacctacc ttcgcgacga 1980
agtagacaaa tacaggatag tcatcggcaa ccagacggta gtctttgaga aagagaagga 2040
tccgtccaag ttacggcgc catctgccgg caaacttatc aatactttgg ttgaagatgg 2100
cggacatgtt gataagggac aaccttacgc tgagattgag gtgatgaaaa tggtgatgac 2160
gctagctgca ccagaatctg gtaaagtgac ctggattctt cgctctggag ccgtgttgga 2220
tatgggagcc atgatcggta ccctggaatt ggacgaccca tcgctcgtga ctactgccat 2280
accgtacaag ggtcagttcc caattgagga caaccagcaa ctttcggaga agctcaacca 2340
tgcacacaac aagtatagag ctgtacttga gaatactctg caaggttact gcctcccaga 2400
gccatacaac acccctcgtc tccgcgaggt ggttgagaag ttcatgcaga gtctacgtga 2460
cccatcgctg ccgttgttgg agctgcagga ggtgctatcg tcgacgtcag gccgtatccc 2520
catagctgtg gagaagaagg ttcgcaagct catggcgttg tacgagagga atatcaccag 2580
cgtgttggcg cagttcccaa gccagcagat agctagtgtt atcgaccacc acgcagcatc 2640
tctagctaag agagccgaca gagatgtgtt cttcatgagc actcaagcgc tcgttgtttt 2700

agtacagaga tatagaaatg gcattcgtgg tagaatgaag gctgccgttc acgatttact 2760
caagcagtac taccaagttg agagtaactt ccaactgggt tcatatgaca aatgcgtggt 2820
ggctctccgc gatcgctata aggacgatat gcaagctgtg tctaacatta tattctcgca 2880
caaccaagtg gctaagaaga atttgttggt aaccctacta atcgaccatc tatggtcgaa 2940
cgagccaggt ctaaccgacg agttggcgac caccctaaac gagctgactt ccttgcacag 3000
agctgaacac agccgagtgg ctctgagagc tagacaggtg ttaatcgcag cacatcaacc 3060
agcctacgag ctacgccaca atcaaatgga atccatattc ttgtcagcag tcgacatgta 3120
tggacatgac ttccacccag agaatctgca gaaactcatt ctctcagaga cttccatttt 3180
cgatattctg cacgacttct tctaccacac taatgctgcg gtatgcaacg cagctttaga 3240
agtatacgtc cgtcgagcgt atacgtcata cgacatcact tgcctccagc acctggcttt 3300
gtctggggag ctgggcgttg tgcacttcca gttcatactg cctactggac atcctaatag 3360
aatcccaatc agccaatcag aaatcgagct agcctccgct tcagaccagg aaggcattcc 3420
ggctgagcta tgcacagcgg ccatgcgcaa atgtcaccac cgcaccggcg cgctggccgc 3480
cttcgagagc ttcgaccagt tcgtgcagta ctctgatgag ctgctcgacc tggtgcacga 3540
cttcgccagc tccgccaccg tcaggagaga ggacttggca gcgttgcagg aaggcagcga 3600
gagtagggat agcaccagca tcaatgtggg aacggacttc aagcctagcg atgctgataa 3660
tgaggccccc ctagaaccga ttcacatact gatgataggc gtacgcgact cgggcgagtc 3720
agacgacagc gcggtgtcgc gccgcttcgg caacttctgt cgcgcgcacc gacacgagct 3780
gcatcagaag agagtgcgac gcattacctt catgctgctt atcaagcgcc aattccccaa 3840
attcttcaca ttccgcgccc gcaacgactt cactgaagac acgatctacc gtcacttgga 3900
gccagcgtcc gccttccagc tggagctgta ccgcatgagg agttacgagc tcgaagcctt 3960
gcctacgagc aaccagaaga tgcacctgta tcttgggaag gctaaggtaa agaagggcca 4020
agaggtgaca gactacaggt tcttcatccg ctccatcatc agacatcagg atctcatcac 4080
caaagaggcc agtttcgagt acctgcagaa tgagggtgaa agggtactct tagaagctat 4140
ggatgaactg gaggtggcct tctcccatcc tttggctaag agaactgatt gcaaccacat 4200
cttcctcaat ttcggaccta ccgtcatcat ggatcctgct aagattgaag aatcagtcct 4260
tggcatggtg atgcgctatg gtcctcgtct atggaaactc agggtactac aagctgaaat 4320
cagattcacg ctgcgtattg gtcccggagc gcccacaaag aacgttcgtc tctgcctggc 4380
gaacggctcc ggctattccc tggacatata cacttatgaa gaggtttccg accccaagat 4440
cggtgtgata atgttccaat ctttcggacc cagacaggga ccgatgcacg gcctaccgat 4500
ctccacacca tatgttacca aagattatct gcagcagaag aggttcttgg ccacatcaca 4560
aggcacgaca tacgtatacg atatcccgga tatgttcaga caaatggtcg agaggagatg 4620
gcgcgagtgc attgaagagg gcagcgttga tggaccgcca ccggataatg tgatgacatc 4680
agtagagctg gtagtggaag ctgatggtga aagacgagtt gtagaagtca ccaggctacc 4740
cggacagaat aacgtcggta tggtagcgtg gcgtttaacc ctgttcacgc cggagtgtcc 4800
cgatggtcgc gacatcatcc tcatagcgaa cgacctcact tactacatgg ggtcgtttgg 4860
accccaagaa gattgggtct attacaaagc ttcggtgtac gcgagggagt tgaagatacc 4920
tagggtatac ataagcgtaa actccggcgc acgtatcgga gttgccgaag aagttaaatc 4980
cgagttcaat gtcgcctggt tagactctga gagaccggac agagggttca aatatctcta 5040
cttgaccccc gagtcatact ctaagctggg accccctgggg tctgtcaaaa ccacgcttat 5100
tgaggacgag ggagagtcca gatataagat caccgatatt attggcaaag aagacggtct 5160
aggcgtggaa tgcctgcgcg acgccggcct catagcgggc gagacggcgc aggcttacga 5220
agatatcgtc accatatcta tcgttacctg ccgagctatc ggcatcggat cttatgtagt 5280
cagattaggc caccgtgtca ttcaagtaga atcttcatac atcattctga cgggttacgc 5340
ggcccttaac aaagtgttgg gacgcgcggt gtacgcgagt aacaaccaac taggggggtca 5400
acaagtcatg catcacaacg gggtctccca cgctgtagcc cccaccgatt tagaggctgt 5460
gaggaccgca ttgagatggc tctcgtttgt gcctaaggac aaattaagca tggtccccat 5520
aatgcgagcg tcggacccca tcgaccggcc cgtggagtgg gccccgcccc gggccgcaca 5580
cgacccgcgc ctcatgctgg ccggggacgc cgccagggcc ggattcttcg acgtcggcag 5640
ctgggatgaa atcatgcagc cttgggcaca gactgttatt actggtcgcg cccgcctggg 5700
tggtatacca gtaggcgtgg tggcagtgga gaccaggact gtagagctga cgctgccagc 5760
tgacccggct aacctggact ccgaagcaaa gaccttgcag caagctggac aggtttggtt 5820
ccccgactcc gcttacaaaa cagctcaagc tatcaacgac ttctcccgcg agaacctgcc 5880
catcatcata ttcgccaact ggagaggatt cagtggtgga cagaaagaca tgtacgaaca 5940
aatcctgaag ttcggtgcgg agatagtccg tgccctacgc ggcgccaccg cccccgtgct 6000
ggtgtacatc ccgcccggcg cggagctgcg cggcggcgcc tgggccgtcg tcgacccctc 6060
cgtcaactcg ctgcgcatgg agatgtatgc tgacccggac gccagaggtg gtgtcttaga 6120
agcggaagcc atcgtggagg tgaagttcaa gcagcgagat atcctcaaaa ccatgcaccg 6180
tttggaccct gaactacaaa ggattggcgc taggatatca gagttgaaag aacaaatcaa 6240
ggagatatca aagagtttgg acagaagagg gtctattgac gagagcctca tcaggaccga 6300
tacaggcagg gcagctgaaa ctcgtgtacg cgaattagaa accgaactat tggcagctga 6360
gaagacagct aaggcacgcg aaaaggaact cagtcctatc tatcaccaga tcgcagtaca 6420
attcgcggaa ctacacgaca ctgcggaaag gatgttagag aaaggatgca tatttgacat 6480
agtaccgtgg cgttcatctc gtaaacagct atactggagg ctgcggcgtc tcctgcgaca 6540
gaacgaacag gagcgacgcg tgcaggcggc cgcccggccc ggacccgcca tgcagcaggg 6600
gcccgccgcc gccacgctgc gcaggtggtt cactgaggac cgcggcgaga cacagtccca 6660
ccagtgggag cacgacaacg aggcagtctg ccgctggcta gaagcacagg ccggagacga 6720
caactccgtg cttgagagga acctccgcgc catccaccaa gacgccttgc tgcaggccgt 6780
caatgattta gtgctggaac tcaccccatc tcagcgatca gaattcatca gaaaactgtc 6840

ggctttagaa atggaacaat agaaagggcg aattcg 6876

<210> 2
<211> 2285
<212> PRT
<213> Heliothis virescens

<400> 2
Met Lys Thr His Arg Thr Phe Tyr Glu Leu Gly Leu Ser Gly Pro Ser
1               5                   10                  15
Met Ser Gln Gly Thr Val Ile His Ser Gln Arg Phe Gln Glu Lys Asp
            20                  25                  30
Phe Thr Val Ala Thr Pro Glu Glu Phe Val Arg Arg Phe Gln Gly Thr
        35                  40                  45
Lys Pro Ile Asn Lys Val Leu Ile Ala Asn Asn Gly Ile Gly Ala Val
    50                  55                  60
Lys Cys Met Arg Ser Ile Arg Arg Trp Ser Tyr Glu Met Phe Lys Asn
65                  70                  75                  80
Glu Arg Ala Val Arg Phe Val Val Met Val Thr Pro Glu Asp Leu Lys
                85                  90                  95
Ala Asn Ala Glu Tyr Ile Lys Met Ala Asp His Tyr Val Pro Val Pro
            100                 105                 110
Gly Gly Ser Asn Asn Asn Asn Tyr Ala Asn Val Glu Leu Ile Val Asp
        115                 120                 125
Ile Ala Ile Arg Thr Gln Val Gln Ala Val Trp Ala Gly Trp Gly His
    130                 135                 140
Ala Ser Glu Asn Pro Lys Leu Pro Glu Leu Leu His Arg Ala Gly Val
145                 150                 155                 160
Val Phe Ile Gly Pro Pro Glu Lys Ala Met Trp Ala Leu Gly Asp Lys
                165                 170                 175
Ile Ala Ser Ser Ile Val Ala Gln Thr Ala Glu Ile Pro Thr Leu Pro
            180                 185                 190
Trp Ser Gly Ser Glu Leu Lys Ala Glu Tyr Asn Ser Lys Lys Ile Lys
        195                 200                 205
Ile Ser Ser Glu Leu Phe Ala Lys Gly Cys Val Thr Thr Pro Glu Gln
    210                 215                 220
Gly Leu Gln Ala Ala Gln Lys Ile Gly Phe Pro Val Met Ile Lys Ala
225                 230                 235                 240
Ser Glu Gly Gly Gly Gly Lys Gly Ile Arg Lys Val Asp Asn Pro Asp
                245                 250                 255
Asp Phe Asn Ser Met Phe Arg Gln Val Gln Ala Glu Val Pro Gly Ser
            260                 265                 270
Pro Ile Phe Val Met Lys Leu Ala Lys Ser Ala Arg His Leu Glu Val
        275                 280                 285
Gln Leu Leu Ala Asp Gln Tyr Gly Asn Ala Ile Ser Leu Phe Gly Arg
    290                 295                 300
Asp Cys Ser Ile Gln Arg Arg His Gln Lys Ile Ile Glu Glu Ala Pro
305                 310                 315                 320
Ala Ala Ile Ala Lys Pro Asp Val Phe Ile Glu Met Glu Lys Ala Ala
                325                 330                 335
Val Arg Leu Ala Lys Met Val Gly Tyr Val Ser Ala Gly Thr Val Glu
            340                 345                 350
Tyr Leu Tyr Glu Pro Ala Thr Gly Ala Tyr Tyr Phe Leu Glu Leu Asn
        355                 360                 365
Pro Arg Leu Gln Val Glu His Pro Cys Thr Glu Met Val Ala Asp Val
    370                 375                 380
Asn Leu Pro Ala Ala Gln Leu Gln Ile Ala Met Gly Leu Pro Leu Tyr
385                 390                 395                 400
His Ile Lys Asp Ile Arg Leu Leu Tyr Gly Glu Ser Pro Trp Gly Leu
                405                 410                 415
Ser Gln Ile Glu Phe Asp Glu Pro Lys Gln Arg Pro Ser Pro Trp Gly
            420                 425                 430
His Val Ile Ala Ala Arg Ile Thr Ser Glu Asn Pro Asp Glu Gly Phe
        435                 440                 445
Lys Pro Ser Ser Gly Thr Val Gln Glu Leu Asn Phe Arg Ser Ser Lys
    450                 455                 460
Asn Val Trp Gly Tyr Phe Ser Val Ala Ala Ser Gly Gly Leu His Glu

465                 470                 475                 480
Phe Ala Asp Ser Gln Phe Gly His Cys Phe Ser Trp Gly Glu Thr Arg
                485                 490                 495
Glu Gln Ala Arg Glu Asn Leu Val Ile Ala Leu Lys Glu Leu Ser Ile
            500                 505                 510
Arg Gly Asp Phe Arg Thr Thr Val Glu Tyr Leu Ile Thr Leu Leu Glu
        515                 520                 525
Thr Gly Ala Phe Gln Asn Asn Asp Ile Asp Thr Ala Trp Leu Asp Ala
    530                 535                 540
Leu Ile Ala Glu Arg Met Gln Ser Glu Lys Pro Asp Ile Met Leu Gly
545                 550                 555                 560
Val Ile Cys Gly Ser Ile Leu Ile Ala Asp Ala Tyr Ile Thr Ala Asn
                565                 570                 575
Phe Gln Glu Phe Lys Ser Ala Leu Glu Lys Gly Gln Ile Gln Gly Ser
            580                 585                 590
Ser Ala Leu Ser Asn Cys Val Glu Val Glu Leu Ile His Ser Gly Ser
        595                 600                 605
Lys Tyr Lys Val Ser Ala Thr Lys Ser Gly Pro Thr Ser Tyr Phe Leu
    610                 615                 620
Ala Met Asn Gly Ser Phe Lys Glu Met Glu Val His Lys Leu Thr Asp
625                 630                 635                 640
Gly Gly Met Leu Leu Ser Ile Asp Gly Ala Ser Tyr Thr Thr Tyr Leu
                645                 650                 655
Arg Asp Glu Val Asp Lys Tyr Arg Ile Val Ile Gly Asn Gln Thr Val
            660                 665                 670
Val Phe Glu Lys Glu Lys Asp Pro Ser Lys Leu Arg Ala Pro Ser Ala
        675                 680                 685
Gly Lys Leu Ile Asn Thr Leu Val Glu Asp Gly Gly His Val Asp Lys
    690                 695                 700
Gly Gln Pro Tyr Ala Glu Ile Glu Val Met Lys Met Val Met Thr Leu
705                 710                 715                 720
Ala Ala Pro Glu Ser Gly Lys Val Thr Trp Ile Leu Arg Ser Gly Ala
                725                 730                 735
Val Leu Asp Met Gly Ala Met Ile Gly Thr Leu Glu Leu Asp Asp Pro
            740                 745                 750
Ser Leu Val Thr Thr Ala Ile Pro Tyr Lys Gly Gln Phe Pro Ile Glu
        755                 760                 765
Asp Asn Gln Gln Leu Ser Glu Lys Leu Asn His Ala His Asn Lys Tyr
    770                 775                 780
Arg Ala Val Leu Glu Asn Thr Leu Gln Gly Tyr Cys Leu Pro Glu Pro
785                 790                 795                 800
Tyr Asn Thr Pro Arg Leu Arg Glu Val Val Glu Lys Phe Met Gln Ser
                805                 810                 815
Leu Arg Asp Pro Ser Leu Pro Leu Leu Glu Leu Gln Glu Val Leu Ser
            820                 825                 830
Ser Thr Ser Gly Arg Ile Pro Ile Ala Val Glu Lys Lys Val Arg Lys
        835                 840                 845
Leu Met Ala Leu Tyr Glu Arg Asn Ile Thr Ser Val Leu Ala Gln Phe
    850                 855                 860
Pro Ser Gln Gln Ile Ala Ser Val Ile Asp His His Ala Ala Ser Leu
865                 870                 875                 880
Ala Lys Arg Ala Asp Arg Asp Val Phe Phe Met Ser Thr Gln Ala Leu
                885                 890                 895
Val Val Leu Val Gln Arg Tyr Arg Asn Gly Ile Arg Gly Arg Met Lys
            900                 905                 910
Ala Ala Val His Asp Leu Leu Lys Gln Tyr Tyr Gln Val Glu Ser Asn
        915                 920                 925
Phe Gln Leu Gly Ser Tyr Asp Lys Cys Val Val Ala Leu Arg Asp Arg
    930                 935                 940
Tyr Lys Asp Asp Met Gln Ala Val Ser Asn Ile Ile Phe Ser His Asn
945                 950                 955                 960
Gln Val Ala Lys Lys Asn Leu Leu Val Thr Leu Leu Ile Asp His Leu
                965                 970                 975
Trp Ser Asn Glu Pro Gly Leu Thr Asp Glu Leu Ala Thr Thr Leu Asn
            980                 985                 990
Glu Leu Thr Ser Leu His Arg Ala Glu His Ser Arg Val Ala Leu Arg
        995                 1000                1005
Ala Arg Gln Val Leu Ile Ala Ala His Gln Pro Ala Tyr Glu Leu Arg
    1010                1015                1020

His Asn Gln Met Glu Ser Ile Phe Leu Ser Ala Val Asp Met Tyr Gly
1025                1030                1035                1040
His Asp Phe His Pro Glu Asn Leu Gln Lys Leu Ile Leu Ser Glu Thr
                1045                1050                1055
Ser Ile Phe Asp Ile Leu His Asp Phe Phe Tyr His Thr Asn Ala Ala
            1060                1065                1070
Val Cys Asn Ala Ala Leu Glu Val Tyr Val Arg Arg Ala Tyr Thr Ser
        1075                1080                1085
Tyr Asp Ile Thr Cys Leu Gln His Leu Ala Leu Ser Gly Glu Leu Gly
    1090                1095                1100
Val Val His Phe Gln Phe Ile Leu Pro Thr Gly His Pro Asn Arg Ile
1105                1110                1115                1120
Pro Ile Ser Gln Ser Glu Ile Glu Leu Ala Ser Ala Ser Asp Gln Glu
                1125                1130                1135
Gly Ile Pro Ala Glu Leu Cys Thr Ala Ala Met Arg Lys Cys His His
            1140                1145                1150
Arg Thr Gly Ala Leu Ala Ala Phe Glu Ser Phe Asp Gln Phe Val Gln
        1155                1160                1165
Tyr Ser Asp Glu Leu Leu Asp Leu Val His Asp Phe Ala Ser Ser Ala
    1170                1175                1180
Thr Val Arg Arg Glu Asp Leu Ala Ala Leu Gln Glu Gly Ser Glu Ser
1185                1190                1195                1200
Arg Asp Ser Thr Ser Ile Asn Val Gly Thr Asp Phe Lys Pro Ser Asp
                1205                1210                1215
Ala Asp Asn Glu Ala Pro Leu Glu Pro Ile His Ile Leu Met Ile Gly
            1220                1225                1230
Val Arg Asp Ser Gly Glu Ser Asp Asp Ser Ala Val Ser Arg Arg Phe
        1235                1240                1245
Gly Asn Phe Cys Arg Ala His Arg His Glu Leu His Gln Lys Arg Val
    1250                1255                1260
Arg Arg Ile Thr Phe Met Leu Leu Ile Lys Arg Gln Phe Pro Lys Phe
1265                1270                1275                1280
Phe Thr Phe Arg Ala Arg Asn Asp Phe Thr Glu Asp Thr Ile Tyr Arg
                1285                1290                1295
His Leu Glu Pro Ala Ser Ala Phe Gln Leu Glu Leu Tyr Arg Met Arg
            1300                1305                1310
Ser Tyr Glu Leu Glu Ala Leu Pro Thr Ser Asn Gln Lys Met His Leu
        1315                1320                1325
Tyr Leu Gly Lys Ala Lys Val Lys Lys Gly Gln Glu Val Thr Asp Tyr
    1330                1335                1340
Arg Phe Phe Ile Arg Ser Ile Ile Arg His Gln Asp Leu Ile Thr Lys
1345                1350                1355                1360
Glu Ala Ser Phe Glu Tyr Leu Gln Asn Glu Gly Glu Arg Val Leu Leu
                1365                1370                1375
Glu Ala Met Asp Glu Leu Glu Val Ala Phe Ser His Pro Leu Ala Lys
            1380                1385                1390
Arg Thr Asp Cys Asn His Ile Phe Leu Asn Phe Gly Pro Thr Val Ile
        1395                1400                1405
Met Asp Pro Ala Lys Ile Glu Glu Ser Val Leu Gly Met Val Met Arg
    1410                1415                1420
Tyr Gly Pro Arg Leu Trp Lys Leu Arg Val Leu Gln Ala Glu Ile Arg
1425                1430                1435                1440
Phe Thr Leu Arg Ile Gly Pro Gly Ala Pro Thr Lys Asn Val Arg Leu
                1445                1450                1455
Cys Leu Ala Asn Gly Ser Gly Tyr Ser Leu Asp Ile Tyr Thr Tyr Glu
            1460                1465                1470
Glu Val Ser Asp Pro Lys Ile Gly Val Ile Met Phe Gln Ser Phe Gly
        1475                1480                1485
Pro Arg Gln Gly Pro Met His Gly Leu Pro Ile Ser Thr Pro Tyr Val
    1490                1495                1500
Thr Lys Asp Tyr Leu Gln Gln Lys Arg Phe Leu Ala Thr Ser Gln Gly
1505                1510                1515                1520
Thr Thr Tyr Val Tyr Asp Ile Pro Asp Met Phe Arg Gln Met Val Glu
                1525                1530                1535
Arg Arg Trp Arg Glu Cys Ile Glu Glu Gly Ser Val Asp Gly Pro Pro
            1540                1545                1550
Pro Asp Asn Val Met Thr Ser Val Glu Leu Val Val Glu Ala Asp Gly
        1555                1560                1565
Glu Arg Arg Val Val Glu Val Thr Arg Leu Pro Gly Gln Asn Asn Val

1570                    1575                    1580
Gly Met Val Ala Trp Arg Leu Thr Leu Phe Thr Pro Glu Cys Pro Asp
1585                1590                1595                1600
Gly Arg Asp Ile Ile Leu Ile Ala Asn Asp Leu Thr Tyr Tyr Met Gly
                1605                1610                1615
Ser Phe Gly Pro Gln Glu Asp Trp Val Tyr Tyr Lys Ala Ser Val Tyr
            1620                1625                1630
Ala Arg Glu Leu Lys Ile Pro Arg Val Tyr Ile Ser Val Asn Ser Gly
        1635                1640                1645
Ala Arg Ile Gly Val Ala Glu Glu Val Lys Ser Glu Phe Asn Val Ala
    1650                1655                1660
Trp Leu Asp Ser Glu Arg Pro Asp Arg Gly Phe Lys Tyr Leu Tyr Leu
1665                1670                1675                1680
Thr Pro Glu Ser Tyr Ser Lys Leu Gly Pro Leu Gly Ser Val Lys Thr
                1685                1690                1695
Thr Leu Ile Glu Asp Glu Gly Glu Ser Arg Tyr Lys Ile Thr Asp Ile
            1700                1705                1710
Ile Gly Lys Glu Asp Gly Leu Gly Val Glu Cys Leu Arg Asp Ala Gly
        1715                1720                1725
Leu Ile Ala Gly Glu Thr Ala Gln Ala Tyr Glu Asp Ile Val Thr Ile
    1730                1735                1740
Ser Ile Val Thr Cys Arg Ala Ile Gly Ile Gly Ser Tyr Val Val Arg
1745                1750                1755                1760
Leu Gly His Arg Val Ile Gln Val Glu Ser Ser Tyr Ile Ile Leu Thr
                1765                1770                1775
Gly Tyr Ala Ala Leu Asn Lys Val Leu Gly Arg Ala Val Tyr Ala Ser
            1780                1785                1790
Asn Asn Gln Leu Gly Gly Gln Gln Val Met His His Asn Gly Val Ser
        1795                1800                1805
His Ala Val Ala Pro Thr Asp Leu Glu Ala Val Arg Thr Ala Leu Arg
    1810                1815                1820
Trp Leu Ser Phe Val Pro Lys Asp Lys Leu Ser Met Val Pro Ile Met
1825                1830                1835                1840
Arg Ala Ser Asp Pro Ile Asp Arg Pro Val Glu Trp Ala Pro Pro Arg
                1845                1850                1855
Ala Ala His Asp Pro Arg Leu Met Leu Ala Gly Asp Ala Ala Arg Ala
            1860                1865                1870
Gly Phe Phe Asp Val Gly Ser Trp Asp Glu Ile Met Gln Pro Trp Ala
        1875                1880                1885
Gln Thr Val Ile Thr Gly Arg Ala Arg Leu Gly Gly Ile Pro Val Gly
    1890                1895                1900
Val Val Ala Val Glu Thr Arg Thr Val Glu Leu Thr Leu Pro Ala Asp
1905                1910                1915                1920
Pro Ala Asn Leu Asp Ser Glu Ala Lys Thr Leu Gln Gln Ala Gly Gln
                1925                1930                1935
Val Trp Phe Pro Asp Ser Ala Tyr Lys Thr Ala Gln Ala Ile Asn Asp
            1940                1945                1950
Phe Ser Arg Glu Asn Leu Pro Ile Ile Ile Phe Ala Asn Trp Arg Gly
        1955                1960                1965
Phe Ser Gly Gly Gln Lys Asp Met Tyr Glu Gln Ile Leu Lys Phe Gly
    1970                1975                1980
Ala Glu Ile Val Arg Ala Leu Arg Gly Ala Thr Ala Pro Val Leu Val
1985                1990                1995                2000
Tyr Ile Pro Pro Gly Ala Glu Leu Arg Gly Gly Ala Trp Ala Val Val
                2005                2010                2015
Asp Pro Ser Val Asn Ser Leu Arg Met Glu Met Tyr Ala Asp Pro Asp
            2020                2025                2030
Ala Arg Gly Gly Val Leu Glu Ala Glu Ala Ile Val Glu Val Lys Phe
        2035                2040                2045
Lys Gln Arg Asp Ile Leu Lys Thr Met His Arg Leu Asp Pro Glu Leu
    2050                2055                2060
Gln Arg Ile Gly Ala Arg Ile Ser Glu Leu Lys Glu Gln Ile Lys Glu
2065                2070                2075                2080
Ile Ser Lys Ser Leu Asp Arg Arg Gly Ser Ile Asp Glu Ser Leu Ile
                2085                2090                2095
Arg Thr Asp Thr Gly Arg Ala Ala Glu Thr Arg Val Arg Glu Leu Glu
            2100                2105                2110
Thr Glu Leu Leu Ala Ala Glu Lys Thr Ala Lys Ala Arg Glu Lys Glu
        2115                2120                2125

Leu Ser Pro Ile Tyr His Gln Ile Ala Val Gln Phe Ala Glu Leu His
    2130                2135                2140
Asp Thr Ala Glu Arg Met Leu Glu Lys Gly Cys Ile Phe Asp Ile Val
2145                2150                2155                2160
Pro Trp Arg Ser Ser Arg Lys Gln Leu Tyr Trp Arg Leu Arg Arg Leu
                2165                2170                2175
Leu Arg Gln Asn Glu Gln Glu Arg Arg Val Gln Ala Ala Ala Arg Pro
            2180                2185                2190
Gly Pro Ala Met Gln Gln Gly Pro Ala Ala Ala Thr Leu Arg Arg Trp
        2195                2200                2205
Phe Thr Glu Asp Arg Gly Glu Thr Gln Ser His Gln Trp Glu His Asp
    2210                2215                2220
Asn Glu Ala Val Cys Arg Trp Leu Glu Ala Gln Ala Gly Asp Asp Asn
2225                2230                2235                2240
Ser Val Leu Glu Arg Asn Leu Arg Ala Ile His Gln Asp Ala Leu Leu
                2245                2250                2255
Gln Ala Val Asn Asp Leu Val Leu Glu Leu Thr Pro Ser Gln Arg Ser
            2260                2265                2270
Glu Phe Ile Arg Lys Leu Ser Ala Leu Glu Met Glu Gln
        2275                2280                2285

## Description

### CROSS-REFERENCE

This application claims the benefit of U. S. Provisional Patent Application No. 60/580,038, filed June 15, 2004, which application is incorporated herein by reference in its entirety.

### FIELD OF THE INVENTION

The invention relates to insect enzymes, and in particular to an insect acetyl CoA carboxylase.

### BACKGROUND OF THE INVENTION

Acetyl CoA carboxylase (ACCase) is the rate limiting enzyme in fatty acid synthesis in most organisms. This enzyme utilizes ATP to charge a biotin functional group with a carboxyl group provided by bicarbonate. The carboxyl group is subsequently transferred to acetyl CoA to yield malonyl CoA.

In prokaryotes and dicotyledenous plants, the enzyme consists of 4 subunits: a biotin carboxylase subunit, a biotin carboxyl carrier protein, and alpha and beta carboxyltransferase proteins. In insects, monocotyledenous plants, and mammals, a single large polypeptide encodes these activities. In wheat, this single polypeptide exists as distinct plastid and cytosolic isoforms of ACCase. In humans, two isoforms of ACCase also exist, alpha which is cytoplasmic and involved in fatty acid synthesis, and beta, which is mitochondrial and involved in beta-oxidation.

Activity of the mammalian cytosolic ACCase is regulated at multiple levels. Allosteric contol is effected by cellular metabolites including glutamate, citrate, and malonyl- and palmitoyl-CoA. Citrate activates the enzyme by polymerization of an inactive protomer to active polymer of approximately 4-8 million Daltons. Conversely, Malonyl CoA or Palmitoyl CoA inhibit the enzyme and promote depolymerization. Glutamate may have both direct allosteric effects as well as indirect effects. ACCase activity is also regulated by phosphorylation.

Several compound classes are known to antagonize ACCase activity. Fibrates are a class of drugs known to affect ACCase activity in mammals and their mechanism of action has been suggested to be mediated via activation of AMP-dependent protein kinase and the subsequent phosphorylation and inactivation of ACCase. Aryloxyphenoxypropionate and cyclohexanedione herbicides inhibit the plastid ACCase of monocots, but not the multisubunit chloroplast enzymes of dicots plants and bacteria or the ACCases from mammals and yeast.

Pesticide development has traditionally focused on the chemical and physical properties of the pesticide itself, a relatively time-consuming and expensive process. As a consequence, efforts have been concentrated on the modification of pre-existing, well-validated compounds, rather than on the development of new pesticides. There is a need in the art for new pesticidal compounds that are safer, more selective, and more efficient than currently available pesticides. The present invention addresses this need by providing novel pesticide targets from invertebrates such as the tobacco budworm *Heliothis virescens,* and by providing methods of identifying compounds that bind to and modulate the activity of such targets.

### Literature

Nikolskaya et al. (1999) *Proc. Natl. Acad. Sci. USA* 96:14647-14651; Munday and Hemingway (1999) *Adv. Enzyme Regul*. 39:205-234; Boone et al. (2000) *J. Biol. Chem.* 275:10819-10825; Zuther et al. (1999) *Proc. Natl. Acad. Sci. USA* 96:13387-13392; Parker et al. (1990) *Proc. Natl. Acad Sci. USA* 87:7175; WO 02/48321.

### SUMMARY OF THE INVENTION

The instant invention provides nucleic acid molecules encoding insect acetyl coenzyme A (CoA) carboxylase (ACCase), as well as ACCase polypeptides encoded thereby. In particular, the invention provides nucleic acids encoding Heliothis ACCase; and ACCase polypeptides encoded thereby. The invention further provides methods of identifying agents that modulate a level of Heliothis acetyl CoA carboxylase mRNA, polypeptide, or enzyme activity. Such agents are candidate insecticidal compounds.

It is an object of the invention to provide isolated insect nucleic acid molecules and proteins that are targets for pesticides. The isolated insect nucleic acid molecules provided herein are useful for producing insect proteins encoded thereby. The insect proteins are useful in assays to identify compounds that modulate a biological activity of the proteins, which assays identify compounds that may have utility as pesticides. It is an object of the present invention to provide invertebrate genes encoding enzymes that can be used in genetic screening methods to characterize pathways that such genes may be involved in, as well as other interacting genetic pathways. It is also an object of the invention to provide methods for screening compounds that interact with a subject invertebrate enzyme. Compounds that interact with a subject invertebrate enzyme may have utility as therapeutics or pesticides.

### BRIEF DESCRIPTIONS OF THE DRAWINGS

Figures 1A-1C provide the nucleotide sequence of a *Heliothis* acetyl CoA carboxylase cDNA (SEQ ID NO:1).

Figure 2 provides the amino acid sequence of a *Heliothis* acetyl CoA carboxylase (SEQ ID NO:2).

### DEFINITIONS

As used herein the term "isolated" is meant to describe a polynucleotide, a polypeptide, an antibody, or a host cell that is in an environment different from that in which the polynucleotide, the polypeptide, the antibody, or the host cell naturally occurs. As used herein, the term "substantially purified" refers to a compound (e.g., either a polynucleotide or a polypeptide or an antibody) that is removed from its natural environment and is at least 60% free, preferably 75% free, and most preferably 90% free from other components with which it is naturally associated.

The terms "polynucleotide" and "nucleic acid molecule," used interchangeably herein, refer to a polymeric forms of nucleotides of any length, either ribonucleotides or deoxynucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups. Alternatively, the backbone of the polynucleotide can comprise a polymer of synthetic subunits such as phosphoramidites and thus can be an oligodeoxynucleoside phosphoramidate or a mixed phosphoramidate-phosphodiester oligomer. Peyrottes et al. (1996) *Nucl. Acids Res.* 24:1841-1848; Chaturvedi et al. (1996) *Nucl. Acids Res.* 24:2318-2323. A polynucleotide may comprise modified nucleotides, such as methylated nucleotides and nucleotide analogs, uracyl, other sugars, and linking groups such as fluororibose and thioate, and nucleotide branches. The sequence of nucleotides may be interrupted by non-nucleotide components. A polynucleotide may be further modified after polymerization, such as by conjugation with a labeling component. Other types of modifications included in this definition are caps, substitution of one or more of the naturally occurring nucleotides with an analog, and introduction of means for attaching the polynucleotide to proteins, metal ions, labeling components, other polynucleotides, or a solid support.

For hybridization probes, it may be desirable to use nucleic acid analogs, in order to improve the stability and binding affinity. A number of modifications have been described that alter the chemistry of the phosphodiester backbone, sugars or heterocyclic bases.

Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include 3'-O-5'-S-phosphorothioate, 3'-S-5'-O-phosphorothioate, 3'-CH₂-5'-O-phosphonate and 3'-NH-5'-O-phosphoroamidate. Peptide nucleic acids replace the entire phosphodiester backbone with a peptide linkage.

Sugar modifications are also used to enhance stability and affinity. The α-anomer of deoxyribose may be used, where the base is inverted with respect to the natural β-anomer. The 2'-OH of the ribose sugar may be altered to form 2'-O-methyl or 2'-O-allyl sugars, which provides resistance to degradation without compromising affinity. Modification of the heterocyclic bases must maintain proper base pairing. Some useful substitutions include deoxyuridine for deoxythymidine; 5-methyl-2'-deoxycytidine and 5-bromo-2'-deoxycytidine for deoxycytidine. 5- propynyl-2'-deoxyuridine and 5-propynyl-2'-deoxycytidine have been shown to increase affinity and biological activity when substituted for deoxythymidine and deoxycytidine, respectively.

The terms "polypeptide" and "protein", used interchangeably herein, refer to a polymeric form of amino acids of any length, which can include coded and non-coded amino acids, chemically or biochemically modified or derivatized amino acids, and polypeptides having modified peptide backbones. The term includes fusion proteins, including, but not limited to, fusion proteins with a heterologous amino acid sequence, fusions with heterologous and homologous leader sequences, with or without N-terminal methionine residues; immunologically tagged proteins; and the like.

A "host cell," as used herein, denotes microorganisms or eukaryotic cells or cell lines cultured as unicellular entities which can be, or have been, used as recipients for recombinant vectors or other transfer polynucleotides, and include the progeny of the original cell which has been transfected. It is understood that the progeny of a single cell may not necessarily be completely identical in morphology or in genomic or total DNA complement as the original parent, due to natural, accidental, or deliberate mutation. A "recombinant host cell" is a host cell into which has been introduced a subject nucleic acid molecule or a subject recombinant vector.

By "transformation" is meant a permanent or transient genetic change induced in a cell following incorporation of new DNA (i.e., DNA exogenous to the cell). Genetic change can be accomplished either by incorporation of the new DNA into the genome of the host cell, or by transient or stable maintenance of the new DNA as an episomal element. Where the cell is a eukaryotic cell, a permanent genetic change is generally achieved by introduction of the DNA into the genome of the cell.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a," "and," and "the" include plural referents unless the context clearly dictates otherwise. Thus, for example, reference to "a pesticidal agent" includes a plurality of such agents and reference to "the acetyl CoA carboxylase" includes reference to one or more such carboxylases and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION OF THE INVENTION

A cDNA encoding a full-length open reading frame of acetyl CoA carboxylase (ACCase) was amplified from a *Heliothis virescens* cDNA library, and sequenced in its entirety.

The present invention provides insect ACCase nucleic acid and protein compositions, as well as methods of identifying agents that modulate the level of insect ACCase mRNA, protein, or enzymatic activity.

### ISOLATED NUCLEIC ACIDS OF THE INVENTION

The invention provides isolated insect nucleic acids comprising nucleotide sequences of invertebrate acetyl CoA carboxylase, particularly nucleic acid sequences of insect acetyl CoA carboxylase, and more particularly nucleic acid sequences of *Heliothis virescens* acetyl CoA carboxylase; compositions comprising the nucleic acids; and methods of using these nucleic acids.

The present invention provides isolated nucleic acid molecules that comprise nucleotide sequences encoding insect proteins that are potential pesticide targets. The isolated nucleic acid molecules have a variety of uses, e.g., as hybridization probes, e.g., to identify nucleic acid molecules that share nucleotide sequence identity; in expression vectors to produce the polypeptides encoded by the nucleic acid molecules; and to modify a host cell or animal for use in assays described hereinbelow.

The term "isolated nucleic acid sequence", as used herein, includes the reverse complement, RNA equivalent, DNA or RNA single- or double-stranded sequences, and DNA/RNA hybrids of the sequence being described, unless otherwise indicated.

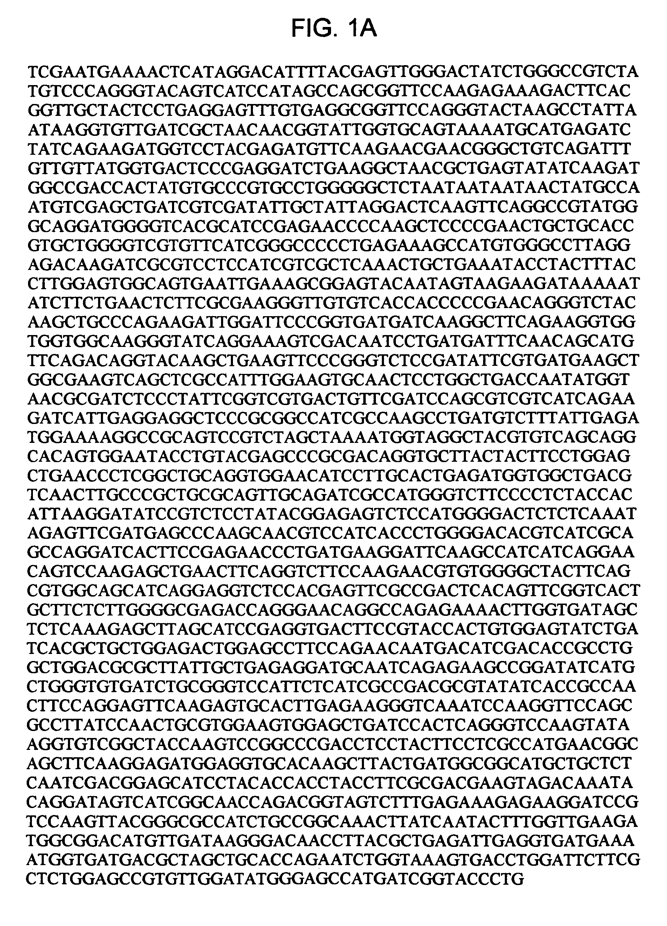

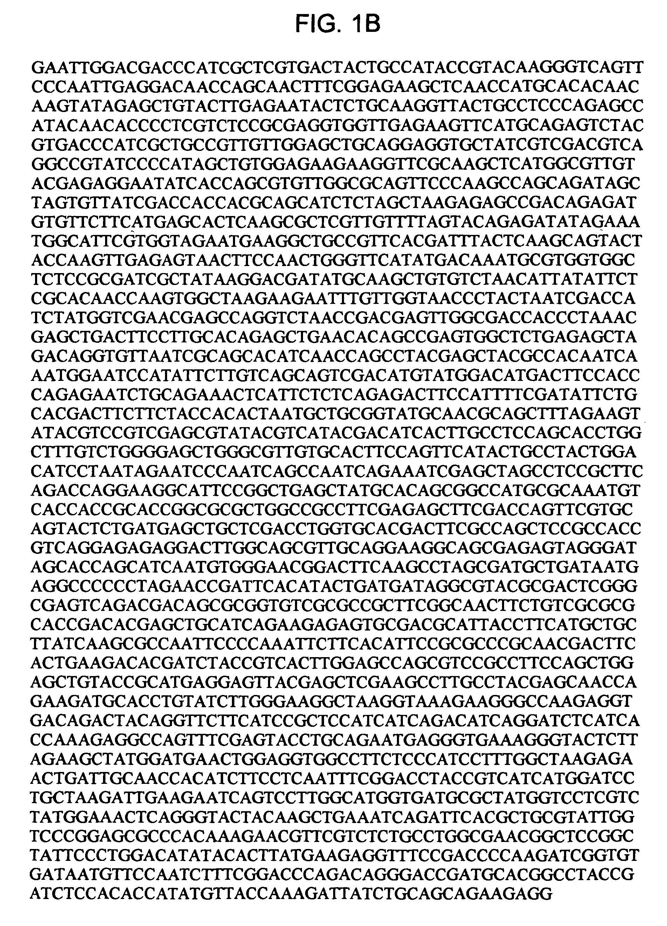

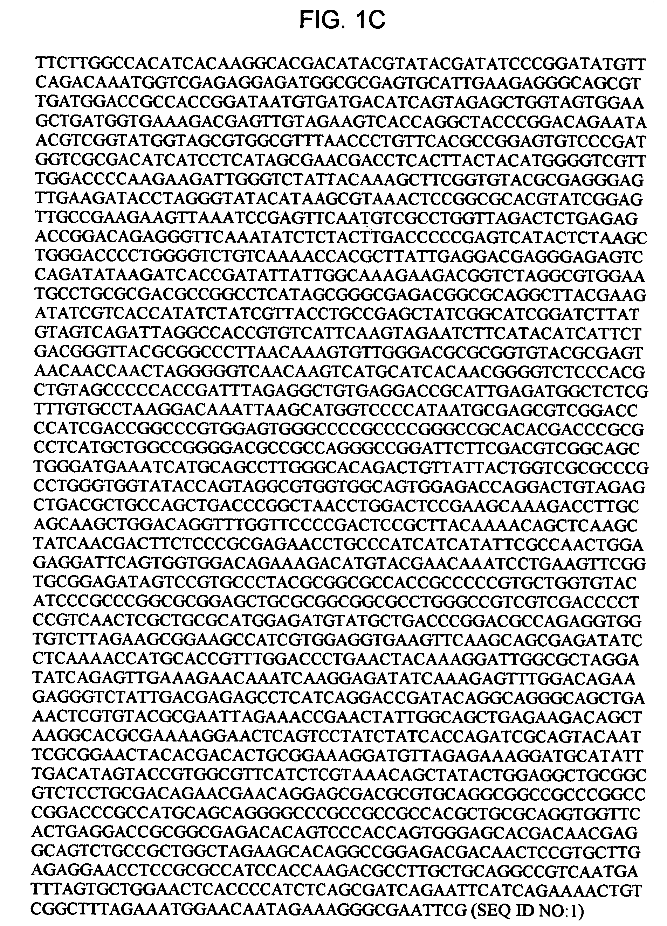

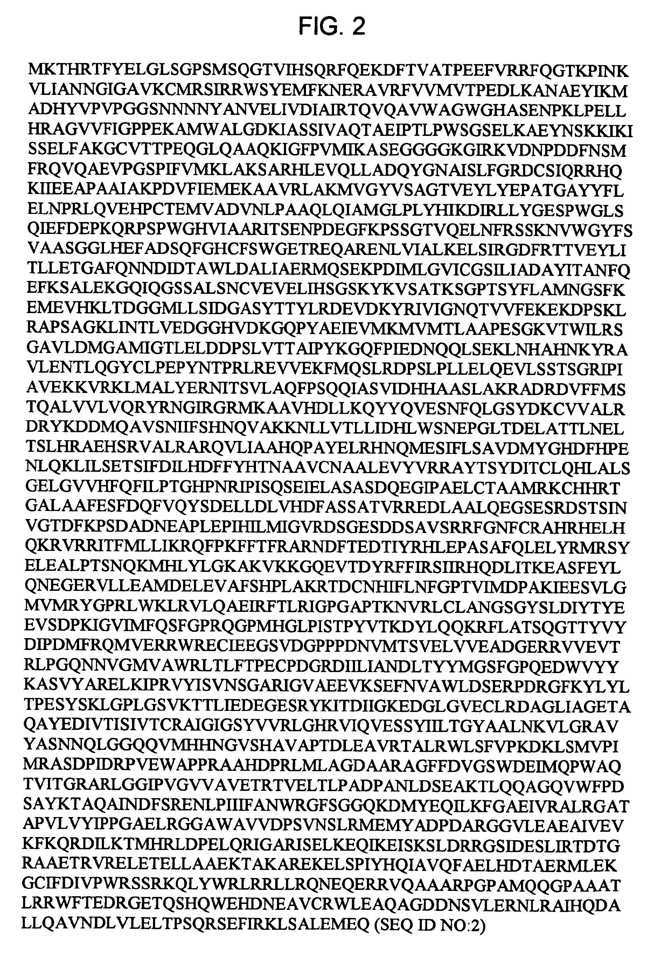

Figures 1A-1C provide the nucleotide sequence (SEQ ID NO: 1) of an acetyl CoA carboxylase from *Heliothis virescens.* Figure 2 provides the amino acid sequence (SEQ ID NO:2) of the encoded *Heliothis virescens* acetyl CoA carboxylase polypeptide.

In some embodiments, an insect acetyl CoA carboxylase nucleic acid comprises a nucleotide sequence having at least about 75%, at least about 80%, at least about 85%, at least about 90%, at least about 95%, at least about 97%, at least about 98%, at least about 99%, or more, nucleotide sequence identity with the sequence set forth in SEQ ID NO: 1. In other embodiments, an insect acetyl CoA carboxylase nucleic acid molecule comprises a nucleotide sequence having the sequence set forth in SEQ ID NO: 1.

In some embodiments, an insect acetyl CoA carboxylase nucleic acid comprises a nucleotide sequence having at least about 75%, at least about 80%, at least about 85%, at least about 90%, at least about 95%, at least about 97%, at least about 98%, at least about 99%, or more, nucleotide sequence identity with the ACCase coding region of SEQ ID NO: 1, e.g., nucleotides5-6859 of SEQ ID NO:1. In other embodiments, an insect acetyl CoA carboxylase nucleic acid molecule comprises a nucleotide sequence having the sequence set forth in nucleotides 5-6859 of SEQ ID NO:1.

In other embodiments, an insect acetyl CoA carboxylase nucleic acid molecule comprises a fragment of at least about 18, at least about 25, at least about 30, at least about 35, at least about 40, at least about 50, at least about 75, at least about 100, at least about 125, at least about 150, at least about 200, at least about 250, at least about 300, at least about 350, at least about 400, at least about 450, at least about 500, at least about 550, at least about 600, at least about 650, at least about 700, at least about 750, at least about 800, at least about 850, at least about 900, at least about 950, at least about 1000, at least about 1100, at least about 1200, at least about 1300, at least about 1400, at least about 1500, at least about 1600, at least about 1700, at least about 1800, at least about 1900, at least about 2000, at least about 3000, at least about 4000, at least about 5000, at least about 6000, or at least about 6800 contiguous nucleotides of nucleotides 5-6859 of the sequence set forth in SEQ ID NO: 1.

In other embodiments, an insect acetyl CoA carboxylase nucleic acid molecule comprises a nucleotide sequence encoding a polypeptide comprising an amino acid sequence having at least about 75%, at least about 80%, at least about 85%, at least about 90%, or at least about 95%, amino acid sequence identity with the amino acid sequence set forth in SEQ ID NO:2. In some embodiments, an insect acetyl CoA carboxylase nucleic acid molecule comprises a nucleotide sequence encoding a polypeptide comprising the sequence set forth in SEQ ID NO:2. In many of these embodiments, the encoded polypeptide has acetyl CoA carboxylase activity.

In other embodiments, an insect acetyl CoA carboxylase nucleic acid molecule comprises a nucleotide sequence encoding a polypeptide comprising a fragment of at least about 6, at least about 10, at least about 15, at least about 20, at least about 25, at least about 30, at least about 40, at least about 50, at least about 75, at least about 100, at least about 125, at least about 150, at least about 175, at least about 200, at least about 225, at least about 250, at least about 275, at least about 300, at least about 325, at least about 350, at least about 375, at least about 400, at least about 425, at least about 450, at least about 475, at least about 500, at least about 525, at least about 550, at least about 600, at least about 700, at least about 800, at least about 900, at least about 1000, at least about 1100, at least about 1200, at least about 1300, at least about 1400, at least about 1500, at least about 1600, at least about 1700, at least about 1800, at least about 1900, at least about 2000, at least about 2100, at least about 2200, or at least about 2275 contiguous amino acids of the sequence set forth in SEQ ID NO:2, up to the entire length of the amino acid sequence set forth in SEQ ID NO:2. In many of these embodiments, the encoded polypeptide has acetyl CoA carboxylase activity.

Fragments of the subject nucleic acid molecules can be used for a variety of purposes. Interfering RNA (RNAi) fragments, particularly double-stranded (ds) RNAi, can be used to generate loss-of-function phenotypes, or to formulate biopesticides (discussed further below). The subject nucleic acid fragments are also useful as nucleic acid hybridization probes and replication/amplification primers. Certain "antisense" fragments, i.e. that are reverse complements of portions of the coding sequence of SEQ ID NO: 1 have utility in inhibiting the function of a subject protein. The fragments are of length sufficient to specifically hybridize with a nucleic acid molecule having the sequence set forth in SEQ ID NO: 1 or nucleotides 5-6859 of the sequence set forth in SEQ ID NO: 1. The fragments consist of or comprise at least 12, preferably at least 24, more preferably at least 36, and more preferably at least 96 contiguous nucleotides of SEQ ID NO: 1 (or of nucleotides 5-6859 of SEQ ID NO: 1). When the fragments are flanked by other nucleic acid sequences, the total length of the combined nucleic acid sequence is less than 15 kb, preferably less than 10 kb or less than 5kb, and more preferably less than 2 kb.

The subject nucleic acid sequences may consist solely of SEQ ID NO: 1 (or of nucleotides 5-6859 of SEQ ID NO:1) or fragments thereof. Alternatively, the subject nucleic acid sequences and fragments thereof may be joined to other components such as labels, peptides, agents that facilitate transport across cell membranes, hybridization-triggered cleavage agents or intercalating agents. The subject nucleic acid sequences and fragments thereof may also be joined to other nucleic acid sequences (i.e. they may comprise part of larger sequences) and are of synthetic/non-natural sequences and/or are isolated and/or are purified, i. e. unaccompanied by at least some of the material with which it is associated in its natural state. Preferably, the isolated nucleic acids constitute at least about 0.5%, and more preferably at least about 5% by weight of the total nucleic acid present in a given fraction, and are preferably recombinant, meaning that they comprise a non-natural sequence or a natural sequence joined to nucleotide(s) other than that which it is joined to on a natural chromosome.

Derivative nucleic acid molecules of the subject nucleic acid molecules include sequences that hybridize to the nucleic acid sequence of SEQ ID NO: 1, or to a nucleic acid molecule containing the open reading frame of SEQ ID NO: 1 (i.e., nucleotides 5-6859 of SEQ ID NO: 1), under stringency conditions such that the hybridizing derivative nucleic acid is related to the subject nucleic acid by a certain degree of sequence identity. A nucleic acid molecule is "hybridizable" to another nucleic acid molecule, such as a cDNA, genomic DNA, or RNA, when a single stranded form of the nucleic acid molecule can anneal to the other nucleic acid molecule. Stringency of hybridization refers to conditions under which nucleic acids are hybridizable. The degree of stringency can be controlled by temperature, ionic strength, pH, and the presence of denaturing agents such as formamide during hybridization and washing. As used herein, the term "stringent hybridization conditions" are those normally used by one of skill in the art to establish at least a 90% sequence identity between complementary pieces of DNA or DNA and RNA. "Moderately stringent hybridization conditions" are used to find derivatives having at least 70% sequence identity. Finally, "low-stringency hybridization conditions" are used to isolate derivative nucleic acid molecules that share at least about 50% sequence identity with the subject nucleic acid sequence.

The ultimate hybridization stringency reflects both the actual hybridization conditions as well as the washing conditions following the hybridization, and it is well known in the art how to vary the conditions to obtain the desired result. Conditions routinely used are set out in readily available procedure texts (e.g., Current Protocol in Molecular Biology, Vol. 1, Chap. 2.10, John Wiley & Sons, Publishers (1994); Sambrook *et al*., Molecular Cloning, Cold Spring Harbor (1989)). In some embodiments, a nucleic acid molecule of the invention is capable of hybridizing to a nucleic acid molecule containing a nucleotide sequence as set forth in SEQ ID NO: 1 (or to nucleotides 5-6859 of SEQ ID NO: 1) under stringent hybridization conditions that comprise: prehybridization of filters containing nucleic acid for 8 hours to overnight at 65° C in a solution comprising 6X single strength citrate (SSC) (1X SSC is 0.15 M NaCl, 0.015 M Na citrate; pH 7.0), 5X Denhardt's solution, 0.05% sodium pyrophosphate and 100 µg/ml herring sperm DNA; hybridization for 18-20 hours at 65° C in a solution containing 6X SSC, 1X Denhardt's solution, 100 µg/ml yeast tRNA and 0.05% sodium pyrophosphate; and washing of filters at 65° C for 1 h in a solution containing 0.2X SSC and 0.1% SDS (sodium dodecyl sulfate).

Derivative nucleic acid sequences that have at least about 75% sequence identity with SEQ ID NO:1 (or to nucleotides 5-6859 of SEQ ID NO: 1) are capable of hybridizing to a nucleic acid molecule containing a nucleotide sequence as set forth in SEQ ID NO: 1 (or to nucleotides 5-6859 of SEQ ID NO: 1) under moderately stringent conditions that comprise: pretreatment of filters containing nucleic acid for 6 h at 40° C in a solution containing 35% formamide, 5X SSC, 50 mM Tris-HCl (pH 7.5), 5 mM EDTA, 0.1% PVP, 0.1% Ficoll, 1% BSA, and 500 µg/ml denatured salmon sperm DNA; hybridization for 18 hours-20 hours at 40° C in a solution containing 35% formamide, 5X SSC, 50 mM Tris-HCl (pH 7.5), 5 mM EDTA, 0.02% PVP, 0.02% Ficoll, 0.2% BSA, 100 µg/ml salmon sperm DNA, and 10% (wt/vol) dextran sulfate; followed by washing twice for 1 hour at 55° C in a solution containing 2X SSC and 0.1% SDS.

Other preferred derivative nucleic acid sequences are capable of hybridizing to SEQ ID NO:1 (or to nucleotides 5-6859 of SEQ ID NO: 1) under low stringency conditions that comprise: incubation for 8 hours to overnight at 37° C in a solution comprising 20% formamide, 5 x SSC, 50 mM sodium phosphate (pH 7.6), 5X Denhardt's solution, 10% dextran sulfate, and 20 µg/ml denatured sheared salmon sperm DNA; hybridization in the same buffer for 18 to 20 hours; and washing of filters in 1 x SSC at about 37° C for 1 hour.

As used herein, "percent (%) nucleic acid sequence identity" with respect to a subject sequence, or a specified portion of a subject sequence, is defined as the percentage of nucleotides in the candidate derivative nucleic acid sequence identical with the nucleotides in the subject sequence (or specified portion thereof), after aligning the sequences and introducing gaps, if necessary to achieve the maximum percent sequence identity, as generated by the program WU-BLAST-2.0a19 (Altschul *et al*., J. Mol. Biol. (1997) 215:403-410; http://blast.wustl.edu/blast/README.html; hereinafter referred to generally as "BLAST") with all the search parameters set to default values. The HSP S and HSP S2 parameters are dynamic values and are established by the program itself depending upon the composition of the particular sequence and composition of the particular database against which the sequence of interest is being searched. A percent (%) nucleic acid sequence identity value is determined by the number of matching identical nucleotides divided by the sequence length for which the percent identity is being reported.

In one preferred embodiment, the derivative nucleic acid encodes a polypeptide comprising an amino acid sequence set forth in SEQ ID NO:2, or a fragment or derivative thereof as described further below. A derivative of a subject nucleic acid molecule, or fragment thereof, may comprise 100% sequence identity with SEQ ID NO:1 (or to nucleotides 5-6859 of SEQ ID NO: 1), but may be a derivative thereof in the sense that it has one or more modifications at the base or sugar moiety, or phosphate backbone. Examples of modifications are well known in the art (Bailey, Ullmann's Encyclopedia of Industrial Chemistry (1998), 6th ed. Wiley and Sons). Such derivatives may be used to provide modified stability or any other desired property.

As used herein, a "derivative" nucleic acid or amino acid sequence includes orthologous sequences of SEQ ID NO:1 (or nucleotides 5-6859 of SEQ ID NO: 1) and SEQ ID NO:2, that are derived from other species. In some embodiments, the orthologue is from a heliothine species, for example *Heliocoverpa armigera* and *Heliothis zea*, which, together with *Heliothis virescens* are three of the world's major crop pests. Orthologous genes of these three species are extremely similar (The International Meeting on Genomics of Lepidoptera, Lyon, France August 16-17, 2001; "International Lepidopteran Genome Project Proposal," Rev. September 10, 2001; available at world wide web site ab.a.u-tokyo.ac.jp/lep-genome/.

In another example, it may be desired to develop a pesticidal agent that specifically targets a non-Heliothine insect species. In such case, it may be most efficient to develop biochemical screening assays (*i*.*e*., assays designed to identify molecules that can inhibit the protein target, as described hereinbelow) using the orthologous protein from that insect. While the orthologues in two species may have essentially the same function, differences in their protein structure may affect properties such as interactions with other proteins, compound binding and stability. Thus, results of a biochemical assays are most meaningful for the specific protein used in the assay. As used herein, orthologues include nucleic acid and polypeptide sequences.

Methods of identifying the orthologues in other species are known in the art. Normally, orthologues in different species retain the same function, due to presence of one or more protein motifs and/or 3-dimensional structures. In evolution, when a gene duplication event follows speciation, a single gene in one species, such as *Heliothis,* may correspond to multiple genes (paralogs) in another. As used herein, the term "orthologues" encompasses paralogs. When sequence data is available for a particular species, orthologues are generally identified by sequence homology analysis, such as BLAST analysis, usually using protein bait sequences. Sequences are assigned as a potential orthologue if the best hit sequence from the forward BLAST result retrieves the original query sequence in the reverse BLAST (Huynen MA and Bork P, Proc Natl Acad Sci (1998) 95:5849-5856; Huynen MA *et al*., Genome Research (2000) 10:1204-1210). Programs for multiple sequence alignment, such as CLUSTAL-W (Thompson JD et al, 1994, Nucleic Acids Res 22:4673-4680) may be used to highlight conserved regions and/or residues of orthologous proteins and to generate phylogenetic trees. In a phylogenetic tree representing multiple homologous sequences from diverse species (e.g., retrieved through BLAST analysis), orthologous sequences from two species generally appear closest on the tree with respect to all other sequences from these two species.

Structural threading or other analysis of protein folding (e.g., using software by ProCeryon, Biosciences, Salzburg, Austria) may also identify potential orthologues. Nucleic acid hybridization methods may also be used to find orthologous genes, e.g., when sequence data are not available. Degenerate PCR and screening of cDNA or genomic DNA libraries are common methods for finding related gene sequences and are well known in the art (see, e.g., Sambrook *et al*. Molecular Cloning: A Laboratory Manual (Second Edition), Cold Spring Harbor Press, Plainview, N.Y., 1989; Dieffenbach C and Dveksler G (Eds.) PCR Primer: A Laboratory Manual, Cold Spring Harbor Laboratory Press, NY, 1989). For instance, methods for generating a cDNA library from an insect species of interest and probing the library with partially homologous gene probes are described in Sambrook *et al*. A highly conserved portion of the *Heliothis* ACCase coding sequence may be used as a probe. ACCase orthologue nucleic acids may hybridize to the nucleic acid of SEQ ID NO: (or nucleotides 5-6859 of SEQ ID NO:1) under high, moderate, or low stringency conditions.

After amplification or isolation of a segment of a putative orthologue, that segment may be cloned and sequenced by standard techniques and utilized as a probe to isolate a complete cDNA or genomic clone. Alternatively, it is possible to initiate an EST project to generate a database of sequence information for the species of interest. In another approach, antibodies that specifically bind known ACCase polypeptides are used for orthologue isolation (Harlow E and Lane D, Antibodies: A Laboratory Manual, Cold Spring Harbor Laboratory Press, 1988, New York; Harlow E and Lane D, Using Antibodies: A Laboratory Manual, Cold Spring Harbor Laboratory Press, 1999, New York).

Western blot analysis can determine that a ACCase orthologue (i.e., an orthologous protein) is present in a crude extract of tissue from a particular species. When reactivity is observed, the sequence encoding the candidate orthologue may be isolated by screening expression libraries representing the particular species. Expression libraries can be constructed in a variety of commercially available vectors, including lambda gt11, as described in Sambrook, *et al*. Once the candidate orthologue(s) are identified by any of these means, candidate orthologous sequence are used as bait (the "query") for the reverse BLAST against sequences from *Heliothis* or other species in which ACCase nucleic acid and/or polypeptide sequences have been identified.

### Isolation, Production, and Expression of Subject Nucleic Acid Molecules

The subject nucleic acids, or fragments or derivatives thereof, may be obtained from an appropriate cDNA library prepared from any eukaryotic species that encodes a subject protein, such as vertebrates, and invertebrates, such as arthropods, particularly insects species (including, but not limited to, *Heliothis), acarids, crustacea, molluscs, nematodes*, and other worms. Where the subject nucleic acid molecule is isolated from a *Heliothis,* any of a variety of field and laboratory strains of various *Heliothis* species can be used, including, but not limited to, *H. virescens, H. maritime, H. ononis, H. peltigera, H. phloxiphaga, H. punctiger, H. subflexa,* and *H. zea*.

An expression library can be constructed using known methods. For example, mRNA can be isolated to make cDNA which is ligated into a suitable expression vector for expression in a host cell into which it is introduced. Various screening assays can then be used to select for the gene or gene product (e.g. oligonucleotides of at least about 20 to 80 bases designed to identify the gene of interest, or labeled antibodies that specifically bind to the gene product). The gene and/or gene product can then be recovered from the host cell using known techniques.

A polymerase chain reaction (PCR) can also be used to isolate a subject nucleic acid molecule, where oligonucleotide primers representing fragmentary sequences of interest amplify RNA or DNA sequences from a source such as a genomic or cDNA library (as described by Sambrook *et al., supra*). Additionally, degenerate primers for amplifying homologs from any species of interest may be used. Once a PCR product of appropriate size and sequence is obtained, it may be cloned and sequenced by standard techniques, and utilized as a probe to isolate a complete cDNA or genomic clone.

Fragmentary sequences of the subject nucleic acid molecules and derivatives thereof may be synthesized by known methods. For example, oligonucleotides may be synthesized using an automated DNA synthesizer available from commercial suppliers (e.g. Biosearch, Novato, CA; Perkin-Elmer Applied Biosystems, Foster City, CA). Antisense RNA sequences can be produced intracellularly by transcription from an exogenous sequence, e.g. from vectors that contain subject antisense nucleic acid sequences. Newly generated sequences may be identified and isolated using standard methods.

An isolated subject nucleic acid molecule can be inserted into any appropriate cloning vector, for example bacteriophages such as lambda derivatives, or plasmids such as pBR322, pUC plasmid derivatives and the Bluescript vector (Stratagene, San Diego, CA). Recombinant molecules can be introduced into host cells via transformation, transfection, infection, electroporation, *etc.,* or into a transgenic animal such as a fly. The transformed cells can be cultured to generate large quantities of the subject nucleic acid. Suitable methods for isolating and producing the subject nucleic acid sequences are well known in the art (Sambrook *et al., supra*; DNA Cloning: A Practical Approach, Vol. 1, 2, 3, 4, (1995) Glover, ed., MRL Press, Ltd., Oxford, U.K.).

The nucleotide sequence encoding a subject protein or fragment or derivative thereof, can be inserted into any appropriate expression vector for the transcription and translation of the inserted protein-coding sequence. Alternatively, the necessary transcriptional and translational signals can be supplied by the native subject gene and/or its flanking regions. A variety of host-vector systems may be utilized to express the protein-coding sequence such as mammalian cell systems infected with virus (e.g. vaccinia virus, adenovirus, *etc.);* insect cell systems infected with virus (e.g. baculovirus); microorganisms such as yeast containing yeast vectors, or bacteria transformed with bacteriophage, DNA, plasmid DNA, or cosmid DNA. Expression of a subject protein may be controlled by a suitable promoter/enhancer element. In addition, a host cell strain may be selected which modulates the expression of the inserted sequences, or modifies and processes the gene product in the specific fashion desired. Preferred host cells include *E. coli,* lepidopteran Sf-9 or S-21 cells, and *Drosophila* S2 cells.

To detect expression of a subject gene product, the expression vector can comprise a promoter operably linked to a subject nucleic acid molecule, one or more origins of replication, and, one or more selectable markers (*e*.*g*. thymidine kinase activity, resistance to antibiotics, *etc.).* Alternatively, recombinant expression vectors can be identified by assaying for the expression of a subject gene product based on the physical or functional properties of a subject protein in *in vitro* assay systems (*e*.*g*. immunoassays).

A subject protein, fragment, or derivative may be optionally expressed as a fusion, or chimeric protein product (i.e. it is joined via a peptide bond to a heterologous protein sequence of a different, i.e., non-acetyl CoA carboxylase protein). In one embodiment, the subject protein is expressed as a fusion protein with a "tag" that facilitates purification, such as glutathione-S-transferase or (His)₆. A chimeric product can be made by ligating the appropriate nucleic acid sequences encoding the desired amino acid sequences to each other in the proper coding frame using standard methods and expressing the chimeric product. A chimeric product may also be made by protein synthetic techniques, e.g. by use of a peptide synthesizer.

Once a recombinant vector that expresses a subject nucleic acid molecule is identified, the encoded subject polypeptide can be isolated and purified using standard methods (e.g. ion exchange, affinity, and gel exclusion chromatography; centrifugation; differential solubility; electrophoresis). The amino acid sequence of the protein can be deduced from the nucleotide sequence of the recombinant nucleic acid molecule contained in the recombinant vector and can thus be synthesized by standard chemical methods (Hunkapiller *et al*., Nature (1984) 310:105-111). Alternatively, native subject proteins can be purified from natural sources, by standard methods (*e*.*g*. immunoaffinity purification).

### RECOMBINANT VECTORS AND HOST CELLS

Also provided are constructs ("recombinant vectors") comprising the subject nucleic acids inserted into a vector, and host cells comprising the constructs. The subject constructs are used for a number of different applications, including propagation, protein production, etc. Viral and non-viral vectors may be prepared and used, including plasmids. The choice of plasmid will depend on the type of cell in which propagation is desired and the purpose of propagation. Certain vectors are useful for amplifying and making large amounts of the desired DNA sequence. Other vectors are suitable for expression in cells in culture. Still other vectors are suitable for transfer and expression in cells in a whole animal. The choice of appropriate vector is well within the skill of the art. Many such vectors are available commercially.

To prepare the constructs, the partial or full-length polynucleotide is inserted into a vector typically by means of DNA ligase attachment to a cleaved restriction enzyme site in the vector. Alternatively, the desired nucleotide sequence can be inserted by homologous recombination *in vivo.* Typically this is accomplished by attaching regions of homology to the vector on the flanks of the desired nucleotide sequence. Regions of homology are added by ligation of oligonucleotides, or by polymerase chain reaction using primers comprising both the region of homology and a portion of the desired nucleotide sequence, for example.

Also provided are expression cassettes or systems that find use in, among other applications, the synthesis of the subject proteins. For expression, the gene product encoded by a polynucleotide of the invention is expressed in any convenient expression system, including, for example, bacterial, yeast, insect, amphibian, and mammalian systems. Suitable vectors and host cells are described in U.S. Patent No. 5,654,173. In the expression vector, an acetyl CoA carboxylase-encoding polynucleotide, e.g., as set forth in SEQ ID NO: 01 (or nucleotides 5-6859 of SEQ ID NO: 1), is operably linked to a regulatory sequence as appropriate to obtain the desired expression properties. These can include promoters (attached either at the 5' end of the sense strand or at the 3' end of the antisense strand), enhancers, terminators, operators, repressors, and inducers. The promoters can be regulated or constitutive. In some situations it may be desirable to use conditionally active promoters, such as tissue-specific, or developmental stage-specific promoters. These are linked to the desired nucleotide sequence using the techniques described above for linkage to vectors. Any techniques known in the art can be used. In other words, the expression vector will provide a transcriptional and translational initiation region, which may be inducible or constitutive, where the coding region is operably linked under the transcriptional control of the transcriptional initiation region, and a transcriptional and translational termination region. These control regions may be native to the subject acetyl CoA carboxylase gene, or may be derived from exogenous sources.

Expression vectors generally have convenient restriction sites located near the promoter sequence to provide for the insertion of nucleic acid sequences encoding heterologous proteins. A selectable marker operative in the expression host may be present, for detection of host cells that comprise the recombinant vector. A variety of markers are known and may be present on the vector, where such markers include those that confer antibiotic resistance, e.g. resistance to ampicillin, tetracycline, chloramphenicol, kanamycin, neomycin; markers that provide for histochemical detection, etc. Expression vectors may be used for, among other things, the production of subject proteins, subject fusion proteins, as described above, and for use in screening assays, as described below.

Expression cassettes may be prepared comprising a transcription initiation region, the gene or fragment thereof, and a transcriptional termination region. Of particular interest is the use of sequences that allow for the expression of functional epitopes or domains, usually at least about 8 amino acids in length, more usually at least about 15 amino acids in length, to about 25 amino acids, and up to the complete open reading frame of the gene. After introduction of the DNA, the cells containing the construct may be selected by means of a selectable marker, the cells expanded and then used for expression.

The above described expression systems may be employed with prokaryotes or eukaryotes in accordance with conventional ways, depending upon the purpose for expression. For large scale production of the protein, or for use in screening assays as described herein, a unicellular organism, such as *E. coli, B. subtilis, S. cerevisiae*, insect cells in combination with baculovirus vectors, or cells of a higher organism such as vertebrates, *e.g.* COS 7 cells, HEK 293, CHO, *Xenopus* oocytes, lepidopteran Sf-9 or S-21 cells, *Drosophila* S2 cells, may be used as the expression host cells. In some situations, it is desirable to express the gene in eukaryotic cells, where the expressed protein will benefit from native folding and post-translational modifications. Small peptides can also be synthesized in the laboratory. Polypeptides that are subsets of the complete protein sequence may be used to identify and investigate parts of the protein important for function.

Specific expression systems of interest include bacterial, yeast, insect cell and mammalian cell derived expression systems. Representative systems from each of these categories is are provided below:

Bacteria. Expression systems in bacteria include those described in Chang *et al., Nature* (1978) *275*:615; Goeddel *et al., Nature* (1979) *281*:544; Goeddel *et al., Nucleic Acids Res.* (1980) *8*:4057; EP 0 036,776; U.S. Patent No. 4,551,433; DeBoer *et al., Proc. Natl. Acad Sci. (USA)* (1983) *80*:21-25; and Siebenlist *et al., Cell* (1980) *20*:269.

Yeast. Expression systems in yeast include those described in Hinnen *et al., Proc. Natl. Acad. Sci. (USA)* (1978) *75*:1929; Ito *et al., J. Bacteriol.* (1983) *153*:163; Kurtz *et al., Mol. Cell. Biol*. (1986) *6*:142; Kunze *et al., J. Basic Microbiol.* (1985) *25*:141; Gleeson *et al., J. Gen. Microbiol.* (1986) *132*:3459; Roggenkamp *et al., Mol. Gen. Genet.* (1986) *202*:302; Das *et al., J. Bacteriol.* (1984) *158*:1165; De Louvencourt *et al., J. Bacteriol.* (1983) *154*:737; Van den Berg *et al., Bio*/*Technology* (1990) *8*:135; Kunze *et al., J. Basic Microbiol.* (1985) *25*:141; Cregg *et al., Mol. Cell. Biol*. (1985) *5*:3376; U.S. Patent Nos. 4,837,148 and 4,929,555; Beach and Nurse, *Nature* (1981) *300*:706; Davidow *et al., Curr. Genet.* (1985) *10*:380; Gaillardin *et al., Curr. Genet.* (1985) *10*:49; Ballance *et al., Biochem. Biophys. Res. Commun.* (1983) *112*:284-289; Tilburn *et al., Gene* (1983) *26*:205-221; Yelton *et al., Proc. Natl. Acad Sci. (USA)* (1984) *81*:1470-1474; Kelly and Hynes, *EMBO J*. (1985) *4*:475479; EP 0 244,234; and WO 91/00357.

Insect Cells. Expression of heterologous genes in insects is accomplished as described in U.S. Patent No. 4,745,051; Friesen *et al*., "The Regulation of Baculovirus Gene Expression", in: *The Molecular Biology Of Baculoviruses* (1986) (W. Doerfler, ed.); EP 0 127,839; EP 0 155,476; and Vlak *et al., J. Gen. Virol*. (1988) *69*:765-776; Miller *et al., Ann. Rev. Microbiol*. (1988) *42*:177; Carbonell *et al., Gene* (1988) *73*:409; Maeda *et al., Nature* (1985) *315*:592-594; Lebacq-Verheyden *et al., Mol. Cell. Biol.* (1988) *8*:3129; Smith *et al., Proc. Natl. Acad. Sci. (USA)* (1985) *82*:8844; Miyajima *et al., Gene* (1987) *58*:273; and Martin *et al., DNA* (1988) *7*:99. Numerous baculoviral strains and variants and corresponding permissive insect host cells from hosts are described in Luckow *et al., Bio*/*Technology* (1988) *6*:47-55, Miller *et al., Generic Engineering* (1986) *8*:277-279, and Maeda *et al., Nature* (1985) *315*:592-594. Various insect cells, including lepidopteran Sf-9 cells and S-21 cells, and *Drosophila* S2 cells, have been amply described in the art. See, e.g., "Insect Cell Culture Engineering", Goosen, Daugulis, and Faulkner, eds. (1993) Marcel Dekker.

Mammalian Cells. Mammalian expression is accomplished as described in *Dijkema et al., EMBO J*. (1985) 4:761, Gorman *et al., Proc. Natl. Acad Sci. (USA)* (1982) 79:6777, Boshart *et al., Cell* (1985) *41*:521 and U.S. Patent No. 4,399,216. Other features of mammalian expression are facilitated as described in Ham and Wallace, *Meth. Enz*. (1979) 58:44, Barnes and Sato, *Anal. Biochem.* (1980) *102*:255, U.S. Patent Nos. 4,767,704, 4,657,866, 4,927,762, 4,560,655, WO 90/103430, WO 87/00195, and U.S. RE 30,985.

Plant cells. Plant cell culture is amply described in various publications, including, e.g., Plant Cell Culture: A Practical Approach, (1995) R.A. Dixon and R. A. Gonzales, eds., IRL Press; and U.S. Patent No. 6,069,009.

Following preparation of the expression vector, the expression vector will be introduced into an appropriate host cell for production of the subject polypeptide, i.e. a host cell will be transformed with the expression vector. Introduction of the recombinant vector into a host cell is accomplished in any convenient manner, including, but not limited to, calcium phosphate precipitation, electroporation, microinjection, use of lipids (e.g., lipofectin), infection, and the like.

When any of the above host cells, or other appropriate host cells or organisms, are used to replicate and/or express the polynucleotides or nucleic acids of the invention, the resulting replicated nucleic acid, RNA, expressed protein or polypeptide, is within the scope of the invention as a product of the host cell or organism. The product is recovered by any appropriate means known in the art.

The invention further provides recombinant host cells, as described above, which contain a subject recombinant vector comprising a subject acetyl CoA carboxylase nucleic acid molecule, e.g., as part of a recombinant vector, either extrachromosomally or integrated into the genome of the host cell. Recombinant host cells are generally isolated, but may also be part of a multicellular organism, e.g., a transgenic animal. Thus, the invention further provides transgenic, non-human animals, particularly insects, that comprise a subject acetyl CoA carboxylase nucleic acid molecule.

The subject nucleic acid molecules can be used to generate transgenic, non-human animals or plants, or site-specific gene modifications in cell lines. Transgenic animals and plants may be made through homologous recombination, where the endogenous locus is altered. Alternatively, a nucleic acid construct is randomly integrated into the genome. Vectors for stable integration include plasmids, retroviruses and other animal viruses, YACs, and the like. Transgenic insects are useful in screening assays, as described below. Insect transgenesis has been described in, e.g., "Insect Transgenesis: Methods and Applications" Handler and James, eds. (2000) CRC Press.

### ISOLATED POLYPEPTIDES

The invention further provides isolated polypeptides comprising or consisting of an amino acid sequence of SEQ ID NO:2, or fragments, variants, or derivatives thereof. Compositions comprising any of these proteins may consist essentially of a subject protein, fragments, or derivatives, or may comprise additional components (e.g. pharmaceutically acceptable carriers or excipients, culture media, carriers used in pesticide formulations, *etc*.).

A derivative of a subject protein typically shares a certain degree of sequence identity or sequence similarity with SEQ ID NO:2, or a fragment thereof As used herein, "percent (%) amino acid sequence identity" with respect to a subject sequence, or a specified portion of a subject sequence, is defined as the percentage of amino acids in the candidate derivative amino acid sequence identical with the amino acid in the subject sequence (or specified portion thereof), after aligning the sequences and introducing gaps, if necessary to achieve the maximum percent sequence identity, as generated by BLAST (Altschul *et al., supra*) using the same parameters discussed above for derivative nucleic acid sequences. A % amino acid sequence identity value is determined by the number of matching identical amino acids divided by the sequence length for which the percent identity is being reported.

"Percent (%) amino acid sequence similarity" is determined by doing the same calculation as for determining % amino acid sequence identity, but including conservative amino acid substitutions in addition to identical amino acids in the computation. A conservative amino acid substitution is one in which an amino acid is substituted for another amino acid having similar properties such that the folding or activity of the protein is not significantly affected. Aromatic amino acids that can be substituted for each other are phenylalanine, tryptophan, and tyrosine; interchangeable hydrophobic amino acids are leucine, isoleucine, methionine, and valine; interchangeable polar amino acids are glutamine and asparagine; interchangeable basic amino acids are arginine, lysine and histidine; interchangeable acidic amino acids are aspartic acid and glutamic acid; and interchangeable small amino acids are alanine, serine, threonine, cysteine, and glycine.

In some embodiments, a subject protein variant or derivative shares at least about 75%, at least 80% sequence identity or similarity, at least 85%, at least 90%, at least about 95%, at least about 97%, at least about 98%, or at least about 99% sequence identity or similarity with a contiguous stretch of at least 25 amino acids, at least 50 amino acids, at least 100 amino acids, at least 200 amino acids, at least 300 amino acids, at least 400 amino acids, at least 500 amino acids, at least about 600, at least about 700, at least about 800, at least about 900, at least about 1000, at least about 1100, at least about 1200, at least about 1300, at least about 1400, at least about 1500, at least about 1600, at least about 1700, at least about 1800, at least about 1900, at least about 2000, at least about 2100, at least about 2000, or at least about 2275 contiguous amino acids of SEQ ID NO:2, and in some cases, the entire length of SEQ ID NO:2. In some embodiments, a polypeptide of the invention comprises an amino acid sequence as set forth in SEQ ID NO:2. In many of these embodiments, the acetyl CoA carboxylase polypeptide has acetyl CoA carboxylase enzyme activity.

In some embodiments, an acetyl CoA carboxylase polypeptide of the invention comprises a fragment of at least about 6, at least about 10, at least about 15, at least about 20, at least about 25, at least about 30, at least about 40, at least about 50, at least about 75, at least about 100, at least about 125, at least about 150, at least about 175, at least 200 amino acids, at least 300 amino acids, at least 400 amino acids, at least 500 amino acids, at least about 600, at least about 700, at least about 800, at least about 900, at least about 1000, at least about 1100, at least about 1200, at least about 1300, at least about 1400, at least about 1500, at least about 1600, at least about 1700, at least about 1800, at least about 1900, at least about 2000, at least about 2100, at least about 2000, or at least about 2275 contiguous amino acids of SEQ ID NO:2, and in some cases, the entire length of SEQ ID NO:2. In many of these embodiments, the acetyl CoA carboxylase polypeptide has acetyl CoA carboxylase enzyme activity.

The fragment or derivative of a subject protein is preferably "functionally active" meaning that the subject protein derivative or fragment exhibits one or more functional activities associated with a full-length, wild-type subject protein comprising the amino acid sequence of SEQ ID NO:2. As one example, a fragment or derivative may have antigenicity such that it can be used in immunoassays, for immunization, for inhibition of activity of a subject protein, etc, as discussed further below regarding generation of antibodies to subject proteins. In many embodiments, a functionally active fragment or derivative of a subject protein is one that displays one or more biological activities associated with a subject protein, such as catalytic activity. For purposes herein, functionally active fragments also include those fragments that exhibit one or more structural features of a subject protein, such as transmembrane or enzymatic domains. Protein domains can be identified using the PFAM program *(see, e.g.,* Bateman A., et al., Nucleic Acids Res, 1999, 27:260-2; and the world wide web at pfam.wustl.edu).

The functional activity of the subject proteins, derivatives and fragments can be assayed by various methods known to one skilled in the art (Current Protocols in Protein Science (1998) Coligan *et al*., eds., John Wiley & Sons, Inc., Somerset, New Jersey).

ACCase catalyzes the ATP- and biotin-dependent formation of malonyl CoA. Enzymatic activity of ACCase can be detected and/or measured using any known assay. As one non-limiting example, ACCase activity can be detected and/or measured using an assay as described in Boone et al. ((2000) *J. Biol. Chem.* 275:10819-10825). For example, a [¹⁴C]biocarbonate fixation method can be used, where formation of [¹⁴C]malonyl CoA is detected using, e.g., high performance liquid chromatography (HPLC). As one non-limiting example, 200 µL of a reaction mixture containing 50 mM HEPES (pH 8), 2.5 mM MgCl₂, 1 mM ATP, 0.5 mM DTT, 10 mM NaHCO₃, 0.95 mM NaH¹⁴CO₃ (487. mCi/mmol), 0.03% (v/v) DMSO and 3-9 µg ACCase is prepared. Acetyl CoA is added to a final concentration of 0.33 mM to start the reaction, and the reaction is allowed to proceed for 10 minutes at 37°C. The reaction is stopped by addition of concentrated HCl, and incorporation of ¹⁴C into malonyl CoA is determined using thin layer chromatography.

A spectrophotometric assay for ACCase enzyme activity can also be used. See, e.g., Weatherly et al. ((2004) *Biochem. J*. 380:105-110) for a description of a suitable spectrophotometric assay. For example, a reaction mixture containing 50 mM HEPES (pH 8), 2.5 mM MgCl₂, 0.5 mM phosphoenolpyruvate, 0.2 mM NADH, 1.1 Units (U) pyruvate kinase, 2.3 U lactate dehydrogenase, 11 mM NaHCO₃, 1 mM ATP, 0.5 mM dithiothreitol, 0.3% (v/v) dimethyl sulfoxide (DMSO), and about 3 µg ACCase is prepared. Acetyl CoA is added to a final concentration of 0.33 mM to start the reaction, and the reaction is allowed to proceed at 30°C for about 10 minutes. The time-dependent decrease in absorbance at 340 nm is measured spectrophotometrically. The decrease in absorbance at 340 nm is proportional to ACCase enzymatic activity.

The subject proteins and polypeptides may be obtained from naturally occurring sources or synthetically produced. For example, wild type proteins may be derived from biological sources which express the proteins, e.g., *Heliothis.* The subject proteins may also be derived from synthetic means, e.g. by expressing a recombinant gene encoding protein of interest in a suitable host, as described above. Any convenient protein purification procedures may be employed, where suitable protein purification methodologies are described in Guide to Protein Purification, (Deuthser ed.) (Academic Press, 1990). For example, a lysate may prepared from the original source and purified using HPLC, exclusion chromatography, gel electrophoresis, affinity chromatography, and the like.

A derivative of a subject protein can be produced by various methods known in the art. The manipulations which result in their production can occur at the gene or protein level. For example, a cloned subject gene sequence can be cleaved at appropriate sites with restriction endonuclease(s) (Wells *et al*., Philos. Trans. R. Soc. London SerA (1986) 317:415), followed by further enzymatic modification if desired, isolated, and ligated *in vitro,* and expressed to produce the desired derivative. Alternatively, a subject gene can be mutated *in vitro or in vivo,* to create and/or destroy translation, initiation, and/or termination sequences, or to create variations in coding regions and/or to form new restriction endonuclease sites or destroy preexisting ones, to facilitate further *in vitro* modification. A variety of mutagenesis techniques are known in the art such as chemical mutagenesis, *in vitro* site-directed mutagenesis (Carter *et al*., Nucl. Acids Res. (1986) 13:4331), use of TAB® linkers (available from Pharmacia and Upjohn, Kalamazoo, MI), *etc.*

At the protein level, manipulations include post translational modification, *e.g.* glycosylation, acetylation, phosphorylation, amidation, derivatization by known protecting/blocking groups, proteolytic cleavage, linkage to an antibody molecule or other cellular ligand, *etc.* Any of numerous chemical modifications may be carried out by known technique (e.g. specific chemical cleavage by cyanogen bromide, trypsin, chymotrypsin, papain, V8 protease, NaBH₄, acetylation, formylation, oxidation, reduction, metabolic synthesis in the presence of tunicamycin, *etc*.). Derivative proteins can also be chemically synthesized by use of a peptide synthesizer, for example to introduce nonclassical amino acids or chemical amino acid analogs as substitutions or additions into the subject protein sequence.

Chimeric or fusion proteins can be made comprising a subject protein or fragment thereof (preferably comprising one or more structural or functional domains of the subject protein) joined at its amino- or carboxy-terminus via a peptide bond to an amino acid sequence of a different protein. Chimeric proteins can be produced by any known method, including: recombinant expression of a nucleic acid encoding the protein (comprising an amino acid sequence encoding a subject protein joined in-frame to a coding sequence for a different protein); ligating the appropriate nucleic acid sequences encoding the desired amino acid sequences to each other in the proper coding frame, and expressing the chimeric product; and protein synthetic techniques, e.g. by use of a peptide synthesizer.

### GENE REGULATORY ELEMENTS OF THE SUBJECT NUCLEIC ACIDS

The invention further provides gene regulatory DNA elements, such as enhancers or promoters that control transcription of the subject nucleic acid molecules. Such regulatory elements can be used to identify tissues, cells, genes and factors that specifically control production of a subject protein. Analyzing components that are specific to a particular subject protein function can lead to an understanding of how to manipulate these regulatory processes, especially for pesticide and therapeutic applications, as well as an understanding of how to diagnose dysfunction in these processes.

Gene fusions with the subject regulatory elements can be made. For compact genes that have relatively few and small intervening sequences, such as those described herein for *Heliothis,* it is typically the case that the regulatory elements that control spatial and temporal expression patterns are found in the DNA immediately upstream of the coding region, extending to the nearest neighboring gene. Regulatory regions can be used to construct gene fusions where the regulatory DNAs are operably fused to a coding region for a reporter protein whose expression is easily detected, and these constructs are introduced as transgenes into the animal of choice.

An entire regulatory DNA region can be used, or the regulatory region can be divided into smaller segments to identify sub-elements that might be specific for controlling expression a given cell type or stage of development. Reporter proteins that can be used for construction of these gene fusions include *E. coli* beta-galactosidase and green fluorescent protein (GFP). These can be detected readily *in situ,* and thus are useful for histological studies and can be used to sort cells that express a subject protein (O'Kane and Gehring PNAS (1987) 84(24):9123-9127; Chalfie *et al*., Science (1994) 263:802-805; and Cumberledge and Krasnow (1994) Methods in Cell Biology 44:143-159). Recombinase proteins, such as FLP or cre, can be used in controlling gene expression through site-specific recombination (Golic and Lindquist (1989) Cell 59(3):499-509; White *et al*., Science (1996) 271:805-807). Toxic proteins such as the reaper and hid cell death proteins, are useful to specifically ablate cells that normally express a subject protein in order to assess the physiological function of the cells (Kingston, In Current Protocols in Molecular Biology (1998) Ausubel *et al*., John Wiley & Sons, Inc. sections 12.0.3-12.10) or any other protein where it is desired to examine the function this particular protein specifically in cells that synthesize a subject protein.

Alternatively, a binary reporter system can be used, similar to that described further below, where a subject regulatory element is operably fused to the coding region of an exogenous transcriptional activator protein, such as the GAL4 or tTA activators described below, to create a subject regulatory element "driver gene". For the other half of the binary system the exogenous activator controls a separate "target gene" containing a coding region of a reporter protein operably fused to a cognate regulatory element for the exogenous activator protein, such as UAS_{G} or a tTA-response element, respectively. An advantage of a binary system is that a single driver gene construct can be used to activate transcription from preconstructed target genes encoding different reporter proteins, each with its own uses as delineated above.

Subject regulatory element-reporter gene fusions are also useful for tests of genetic interactions, where the objective is to identify those genes that have a specific role in controlling the expression of subject genes, or promoting the growth and differentiation of the tissues that expresses a subject protein. Subject gene regulatory DNA elements are also useful in protein-DNA binding assays to identify gene regulatory proteins that control the expression of subject genes. The gene regulatory proteins can be detected using a variety of methods that probe specific protein-DNA interactions well known to those skilled in the art (Kingston, *supra*) including *in vivo* footprinting assays based on protection of DNA sequences from chemical and enzymatic modification within living or permeabilized cells; and *in vitro* footprinting assays based on protection of DNA sequences from chemical or enzymatic modification using protein extracts, nitrocellulose filter-binding assays and gel electrophoresis mobility shift assays using radioactively labeled regulatory DNA elements mixed with protein extracts. Candidate gene regulatory proteins can be purified using a combination of conventional and DNA-affinity purification techniques. Molecular cloning strategies can also be used to identify proteins that specifically bind subject gene regulatory DNA elements. For example, a *Drosophila* cDNA library in an expression vector, can be screened for cDNAs that encode subject gene regulatory element DNA-binding activity. Similarly, the yeast "one-hybrid" system can be used (Li and Herskowitz, Science (1993) 262:1870-1874; Luo *et al., Biotechniques* (1996) 20(4):564-568; Vidal *et al., Proc. Natl. Acad. Sci. USA* (1996) 93(19):10315-10320).

### ANTIBODIES SPECIFIC FOR SUBJECT PROTEINS

The present invention provides antibodies, which may be isolated antibodies, which bind specifically to a subject protein; and compositions comprising the antibodies. The subject proteins, fragments thereof, and derivatives thereof may be used as an immunogen to generate monoclonal or polyclonal antibodies and antibody fragments or derivatives (e.g. chimeric, single chain, Fab fragments). As used herein, the term "antibodies" includes antibodies of any isotype, fragments of antibodies which retain specific binding to antigen, including, but not limited to, Fab, Fv, scFv, and Fd fragments, chimeric antibodies, humanized antibodies, single-chain antibodies, and fusion proteins comprising an antigen-binding portion of an antibody and a non-antibody protein. Also provided are "artificial" antibodies, e.g., antibodies and antibody fragments produced and selected *in vitro.* In some embodiments, such antibodies are displayed on the surface of a bacteriophage or other viral particle. In many embodiments, such artificial antibodies are present as fusion proteins with a viral or bacteriophage structural protein, including, but not limited to, M13 gene III protein. Methods of producing such artificial antibodies are well known in the art. See, e.g., U.S. Patent Nos. 5,516,637; 5,223,409; 5,658,727; 5,667,988; 5,498,538; 5,403,484; 5,571,698; and 5,625,033.

The antibodies may be detectably labeled, e.g., with a radioisotope, an enzyme which generates a detectable product, a green fluorescent protein, and the like. The antibodies may be further conjugated to other moieties, such as members of specific binding pairs, e.g., biotin (member of biotin-avidin specific binding pair), and the like. The antibodies may also be bound to a solid support, including, but not limited to, polystyrene plates or beads, and the like. For example, fragments of a subject protein, e.g., those identified as hydrophilic, are used as immunogens for antibody production using art-known methods such as by hybridomas; production of monoclonal antibodies in germ-free animals (PCT/US90/02545); the use of human hybridomas (Cole *et al., Proc. Natl. Acad. Sci. USA* (1983) 80:2026-2030; Cole *et al*., in Monoclonal Antibodies and Cancer Therapy (1985) Alan R. Liss, pp. 77-96), and production of humanized antibodies (Jones *et al*., Nature (1986) 321:522-525; U.S. Pat. 5,530,101). In a particular embodiment, subject polypeptide fragments provide specific antigens and/or immunogens, especially when coupled to carrier proteins. For example, peptides are covalently coupled to keyhole limpet antigen (KLH) and the conjugate is emulsified in Freund's complete adjuvant. Laboratory animals, e.g., mice, rats, or rabbits are immunized according to conventional protocol and bled. The presence of specific antibodies is assayed by solid phase immunosorbent assays using immobilized corresponding polypeptide. Specific activity or function of the antibodies produced may be determined by convenient *in vitro,* cell-based, or *in vivo* assays: *e.g. in vitro* binding assays, *etc.* Binding affinity may be assayed by determination of equilibrium constants of antigen-antibody association (usually at least about 10⁷M⁻¹, preferably at least about 10⁸M⁻¹, more preferably at least about 10⁹M⁻¹).

### SCREENING METHODS

The present invention further provides methods of identifying agents that reduce an enzymatic activity of a subject acetyl CoA carboxylase, that reduce the level of acetyl CoA carboxylase mRNA and/or polypeptide levels in a cell, particularly an insect cell. The invention further provides methods for identifying molecules that interact with a subject acetyl CoA carboxylase.

### Methods for identifying molecules that interact with a subject protein

A variety of methods can be used to identify or screen for molecules, such as proteins or other molecules, which interact with a subject protein, or derivatives or fragments thereof. The assays may employ purified protein, or cell lines or model organisms such as *Heliothis, Drosophila,* and *C. elegans*, which have been genetically engineered to express a subject protein. Suitable screening methodologies are well known in the art to test for proteins and other molecules that interact with a subject gene and protein (see e.g., PCT International Publication No. WO 96/34099). The newly identified interacting molecules may provide new targets for pharmaceutical or pesticidal agents. Any of a variety of exogenous molecules, both naturally occurring and/or synthetic (e.g., libraries of small molecules or peptides, or phage display libraries), may be screened for binding capacity. In a typical binding experiment, a subject protein or fragment is mixed with candidate molecules under conditions conducive to binding, sufficient time is allowed for any binding to occur, and assays are performed to test for bound complexes.

Assays to find interacting proteins can be performed by any method known in the art, for example, immunoprecipitation with an antibody that binds to the protein in a complex followed by analysis by size fractionation of the immunoprecipitated proteins (e.g. by denaturing or nondenaturing polyacrylamide gel electrophoresis), Western analysis, nondenaturing gel electrophoresis, two-hybrid systems (Fields and Song, Nature (1989) 340:245-246; U.S. Pat. NO. 5,283,173; for review see Brent and Finley, Annu. Rev. Genet. (1977) 31:663-704), *etc.*

### Immunoassays

Immunoassays can be used to identify proteins that interact with or bind to a subject protein. Various assays are available for testing the ability of a protein to bind to or compete with binding to a wild-type subject protein or for binding to an anti- subject protein antibody. Suitable assays include radioimmunoassays, ELISA (enzyme linked immunosorbent assay), immunoradiometric assays, gel diffusion precipitin reactions, immunodiffusion assays, *in situ* immunoassays (*e.g.,* using colloidal gold, enzyme or radioisotope labels), western blots, precipitation reactions, agglutination assays (e.g., gel agglutination assays, hemagglutination assays), complement fixation assays, immunofluorescence assays, protein A assays, immunoelectrophoresis assays, *etc.*

One or more of the molecules in the immunoassay may be joined to a label, where the label can directly or indirectly provide a detectable signal. Various labels include radioisotopes, fluorescers, chemiluminescers, enzymes, specific binding molecules, particles, e.g. magnetic particles, and the like. Specific binding molecules include pairs, such as biotin and streptavidin, digoxin and antidigoxin etc. For the specific binding members, the complementary member would normally be labeled with a molecule that provides for detection, in accordance with known procedures.

### Identification of Potential Pesticide or Drug Targets

The present invention further provides methods of identifying agents that reduce an enzymatic activity of a subject acetyl CoA carboxylase, that reduce the level of acetyl CoA carboxylase mRNA and/or polypeptide levels in a cell, particularly an insect cell.

Once new target genes or target interacting genes are identified, they can be assessed as potential pesticide or drug targets, or as potential biopesticides. Further, transgenic plants that express subject proteins can be tested for activity against insect pests (Estruch *et al*., Nat. Biotechnol (1997) 15(2):137-141).

As used herein, the term "pesticide" refers generally to chemicals, biological agents, and other compounds that adversely affect insect viability, e.g., that kill, paralyze, sterilize or otherwise disable pest species in the areas of agricultural crop protection, human and animal health. Exemplary pest species include parasites and disease vectors such as mosquitoes, fleas, ticks, parasitic nematodes, chiggers, mites, *etc.* Pest species also include those that are eradicated for aesthetic and hygienic purposes (*e*.*g*. ants, cockroaches, clothes moths, flour beetles, *etc.),* home and garden applications, and protection of structures (including wood boring pests such as termites, and marine surface fouling organisms).

Pesticidal compounds can include traditional small organic molecule pesticides (typified by compound classes such as the organophosphates, pyrethroids, carbamates, and organochlorines, benzoylureas, *etc.).* Other pesticides include proteinaceous toxins such as the *Bacillus thuringiensis* crytoxins (Gill *et al*., Annu Rev Entomol (1992) 37:615-636) and *Photorabdus luminescens* toxins (Bowden *et al*., Science (1998) 280:2129-2132); and nucleic acids such as subject dsRNA or antisense nucleic acids that interfere with activity of a subject nucleic acid molecule.

The terms "candidate agent," "agent", "substance" and "compound" are used interchangeably herein. Candidate agents encompass numerous chemical classes, typically synthetic, semi-synthetic, or naturally-occurring inorganic or organic molecules. Candidate agents may be small organic compounds having a molecular weight of more than 50 and less than about 2,500 daltons. Candidate agents may comprise functional groups necessary for structural interaction with proteins, particularly hydrogen bonding, and may include at least an amine, carbonyl, hydroxyl or carboxyl group, and may contain at least two of the functional chemical groups. The candidate agents may comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof.

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

Candidate agents that reduce an acetyl CoA carboxylase activity of a subject polypeptide, and/or that reduce a level of acetyl CoA carboxylase mRNA and/or polypeptide by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or more, are candidate pesticides.

Candidate agents that reduce acetyl CoA carboxylase activity of a subject acetyl CoA carboxylase and/or that reduce a level of acetyl CoA carboxylase mRNA and/or polypeptide are further tested for toxicity toward vertebrate species, such as mammalian species, etc.; and for bioavailability.

A variety of other reagents may be included in the screening assay. These include reagents like salts, neutral proteins, e.g. albumin, detergents, etc that are used to facilitate optimal protein-protein binding and/or reduce non-specific or background interactions. Reagents that improve the efficiency of the assay, such as protease inhibitors, nuclease inhibitors, anti-microbial agents, etc. may be used. The components are added in any order that provides for the requisite activity. Incubations are performed at any suitable temperature, typically between 4°C and 40°C. Incubation periods are selected for optimum activity, but may also be optimized to facilitate rapid high-throughput screening. Typically between 0.1 and 1 hour will be sufficient.

### Assays of Compounds on purified acetyl CoA carboxylase

The invention provides methods of screening for agents that modulate an enzymatic activity of a subject acetyl CoA carboxylase. Such agents are useful as pesticidal agents. Acetyl CoA carboxylase enzymatic activity is measured as described above, using, e.g., a spectrophotometric assay, following a decrease in absorbance at 340 nm as a measure of ACCase activity.

The present invention provides methods of identifying agents which modulate an enzymatic activity of an acetyl CoA carboxylase polypeptide of the invention. The term "modulate" encompasses an increase or a decrease in the measured acetyl CoA carboxylase activity when compared to a suitable control.

The method generally comprises: a) contacting a test agent with a sample containing an acetyl CoA carboxylase polypeptide; and b) assaying an acetyl CoA carboxylase activity of the acetyl CoA carboxylase polypeptide in the presence of the test agent. An increase or a decrease in acetyl CoA carboxylase activity in comparison to acetyl CoA carboxylase activity in a suitable control (e.g., a sample comprising an acetyl CoA carboxylase polypeptide in the absence of the agent being tested) is an indication that the agent modulates an enzymatic activity of the acetyl CoA carboxylase.

An "agent which modulates an acetyl CoA carboxylase activity of an acetyl CoA carboxylase polypeptide", as used herein, describes any molecule, e.g. synthetic or natural organic or inorganic compound, protein or pharmaceutical, with the capability of altering an acetyl CoA carboxylase activity of an acetyl CoA carboxylase polypeptide, as described herein. Generally a plurality of assay mixtures is run in parallel with different agent concentrations to obtain a differential response to the various concentrations. Typically, one of these concentrations serves as a negative control, i.e. at zero concentration or below the level of detection.

### Assays of Compounds on Insects

Potential insecticidal compounds can be administered to insects in a variety of ways, including orally (including addition to synthetic diet, application to plants or prey to be consumed by the test organism), topically (including spraying, direct application of compound to animal, allowing animal to contact a treated surface), or by injection. Insecticides are typically very hydrophobic molecules and must commonly be dissolved in organic solvents, which are allowed to evaporate in the case of methanol or acetone, or at low concentrations can be included to facilitate uptake (ethanol, dimethyl sulfoxide).

The first step in an insect assay is usually the determination of the minimal lethal dose (MLD) on the insects after a chronic exposure to the compounds. The compounds are usually diluted in DMSO, and applied to the food surface bearing 0-48 hour old embryos and larvae. In addition to MLD, this step allows the determination of the fraction of eggs that hatch, behavior of the larvae, such as how they move /feed compared to untreated larvae, the fraction that survive to pupate, and the fraction that eclose (emergence of the adult insect from puparium). Based on these results more detailed assays with shorter exposure times may be designed, and larvae might be dissected to look for obvious morphological defects. Once the MLD is determined, more specific acute and chronic assays can be designed.

In a typical acute assay, compounds are applied to the food surface for embryos, larvae, or adults, and the animals are observed after 2 hours and after an overnight incubation. For application on embryos, defects in development and the percent that survive to adulthood are determined. For larvae, defects in behavior, locomotion, and molting may be observed. For application on adults, defects in levels and/or enzyme activity are observed, and effects on behavior and/or fertility are noted.

For a chronic exposure assay, adults are placed on vials containing the compounds for 48 hours, then transferred to a clean container and observed for fertility, defects in levels and/or activity of a subject enzyme, and death.

### Assay of Compounds using Cell Cultures

Compounds that modulate (*e*.*g*. block or enhance) a subject protein's activity and/or that modulate a level of acetyl CoA carboxylase mRNA or polypeptide may also be assayed using cell culture. Exemplary cells are cultured insect cells such as *Drosophila* S2 cells. In some embodiments, a recombinant vector that includes a sequence that encodes all or part of a subject acetyl CoA carboxylase is introduced into cells in *in vitro* culture, and the resulting recombinant host cells are used to screen test agents. For example, various compounds added to cells expressing a subject protein may be screened for their ability to modulate the activity of subject genes based upon measurements of a biological activity of a subject protein. For example, compounds may be screened for their ability to modulate the activity of acetyl CoA carboxylase genes based on measurements of acetyl CoA carboxylase activity, acetyl CoA carboxylase mRNA levels or acetyl CoA carboxylase polypeptide levels.

Assays for changes in a biological activity of a subject protein can be performed on cultured cells expressing endogenous normal or mutant subject protein. Such studies also can be performed on cells transfected with vectors capable of expressing the subject protein, or functional domains of one of the subject protein, in normal or mutant form. In addition, to enhance the signal measured in such assays, cells may be cotransfected with nucleic acid molecules, or a subject recombinant vector, encoding a subject protein.

Alternatively, cells expressing a subject protein may be lysed, the subject protein purified, and tested *in vitro* using methods known in the art (Kanemaki M., et al., J Biol Chem, (1999) 274:22437-22444).

A wide variety of cell-based assays may be used for identifying agents which modulate levels of acetyl CoA carboxylase mRNA, for identifying agents that modulate the level of acetyl CoA carboxylase polypeptide, and for identifying agents that modulate the level of acetyl CoA carboxylase activity in a eukaryotic cell, using, for example, an insect cell (e.g., *Drosophila* S2 cells) transformed with a construct comprising a acetyl CoA carboxylase-encoding cDNA such that the cDNA is expressed, or, alternatively, a construct comprising an acetyl CoA carboxylase promoter operably linked to a reporter gene.

Accordingly, the present invention provides a method for identifying an agent, particularly a biologically active agent, that modulates a level of acetyl CoA carboxylase expression in a cell, the method comprising: combining a candidate agent to be tested with a cell comprising a nucleic acid which encodes an acetyl CoA carboxylase polypeptide; and determining the effect of said agent on acetyl CoA carboxylase expression (e.g., determining the effect of the agent on a level of acetyl CoA carboxylase mRNA, a level of acetyl CoA carboxylase polypeptide, or a level of acetyl CoA carboxylase enzyme activity in the cell).

"Modulation" of acetyl CoA carboxylase expression levels includes increasing the level and decreasing the level of acetyl CoA carboxylase mRNA and/or acetyl CoA carboxylase polypeptide encoded by the acetyl CoA carboxylase polynucleotide and/or the level of acetyl CoA carboxylase activity when compared to a control lacking the agent being tested. An increase or decrease of about 1.25-fold, usually at least about 1.5-fold, usually at least about 2-fold, usually at least about 5-fold, usually at least about 10-fold or more, in the level (i.e., an amount) of acetyl CoA carboxylase mRNA and/or polypeptide and/or acetyl CoA carboxylase enzyme activity following contacting the cell with a candidate agent being tested, compared to a control to which no agent is added, is an indication that the agent modulates acetyl CoA carboxylase mRNA levels, acetyl CoA carboxylase polypeptide levels, or acetyl CoA carboxylase enzyme activity in the cell. Of particular interest in many embodiments are candidate agents that reduce a level of acetyl CoA carboxylase mRNA, and/or reduce a level of acetyl CoA carboxylase polypeptide, and/or reduce a level of acetyl CoA carboxylase enzyme activity in an insect cell.

Acetyl CoA carboxylase mRNA and/or polypeptide whose levels or activity are being measured can be encoded by an endogenous acetyl CoA carboxylase polynucleotide, or the acetyl CoA carboxylase polynucleotide can be one that is comprised within a recombinant vector and introduced into the cell, i.e., the acetyl CoA carboxylase mRNA and/or polypeptide can be encoded by an exogenous acetyl CoA carboxylase polynucleotide. For example, a recombinant vector may comprise an isolated acetyl CoA carboxylase transcriptional regulatory sequence, such as a promoter sequence, operably linked to a reporter gene (e.g,. β-galactosidase, chloramphenicol acetyl transferase, horse radish peroxidase, luciferase, green fluorescent protein, or other gene whose product can be easily assayed). In these embodiments, the method for identifying an agent that modulates a level of acetyl CoA carboxylase expression in a cell, comprises: combining a candidate agent to be tested with a cell comprising a nucleic acid which comprises an acetyl CoA carboxylase gene transcriptional regulatory element operably linked to a reporter gene; and determining the effect of said agent on reporter gene expression.

A recombinant vector may comprise an isolated acetyl CoA carboxylase transcriptional regulatory sequence, such as a promoter sequence, operably linked to sequences coding for an acetyl CoA carboxylase polypeptide; or the transcriptional control sequences can be operably linked to coding sequences for an acetyl CoA carboxylase fusion protein comprising acetyl CoA carboxylase polypeptide fused to a polypeptide which facilitates detection. In these embodiments, the method comprises combining a candidate agent to be tested with a cell comprising a nucleic acid which comprises an acetyl CoA carboxylase gene transcriptional regulatory element operably linked to an acetyl CoA carboxylase polypeptide-coding sequence; and determining the effect of said agent on acetyl CoA carboxylase expression, which determination can be carried out by measuring an amount of acetyl CoA carboxylase mRNA, acetyl CoA carboxylase polypeptide, acetyl CoA carboxylase fusion polypeptide, or acetyl CoA carboxylase enzyme activity produced by the cell.

Cell-based assays generally comprise the steps of contacting the cell with an agent to be tested, forming a test sample, and, after a suitable time, assessing the effect of the agent on acetyl CoA carboxylase mRNA levels, acetyl CoA carboxylase polypeptide and/or enzyme levels. A control sample comprises the same cell without the candidate agent added. Acetyl CoA carboxylase expression levels are measured in both the test sample and the control sample. A comparison is made between acetyl CoA carboxylase expression level in the test sample and the control sample. Acetyl CoA carboxylase expression can be assessed using conventional assays. For example, when a cell line is transformed with a construct that results in expression of acetyl CoA carboxylase, acetyl CoA carboxylase mRNA levels can be detected and measured, or acetyl CoA carboxylase polypeptide levels, and/or acetyl CoA carboxylase enzyme levels can be detected and measured. A suitable period of time for contacting the agent with the cell can be determined empirically, and is generally a time sufficient to allow entry of the agent into the cell and to allow the agent to have a measurable effect on acetyl CoA carboxylase mRNA and/or polypeptide levels and/or enzyme activity. Generally, a suitable time is between 10 minutes and 24 hours, e.g., about 1 hour to 8 hours.

Methods of measuring acetyl CoA carboxylase mRNA levels are known in the art, several of which have been described above, and any of these methods can be used in the methods of the present invention to identify an agent which modulates acetyl CoA carboxylase mRNA level in a cell, including, but not limited to, a PCR, such as a PCR employing detectably labeled oligonucleotide primers, and any of a variety of hybridization assays. Similarly, acetyl CoA carboxylase polypeptide levels can be measured using any standard method, several of which have been described herein, including, but not limited to, an immunoassay such as an enzyme-linked immunosorbent assay (ELISA), for example an ELISA employing a detectably labeled antibody specific for an acetyl CoA carboxylase polypeptide. Acetyl CoA carboxylase enzyme activity can be measured as described above.

Compounds that selectively modulate a level of a subject acetyl CoA carboxylase-encoding nucleic acid molecule, or that selectively modulate a level of a subject protein, or that selectively modulates a level of acetyl CoA carboxylase enzyme activity, are identified as potential pesticide and drug candidates having specificity for the subject protein. Whether a candidate compound selectively modulates a level of a subject acetyl CoA carboxylase-encoding nucleic acid molecule, or selectively modulates a level of a subject protein, or selectively modulates a level of acetyl CoA carboxylase enzyme activity can be determined by measuring the level of an mRNA or protein, e.g., GAPDH, or other suitable control protein or mRNA, where a candidate agent is "selective" if it does not substantially inhibit the production of or activity of any protein or mRNA other than an acetyl CoA carboxylase protein or acetyl CoA carboxylase-encoding mRNA.

Identification of small molecules and compounds as potential pesticides or pharmaceutical compounds from large chemical libraries requires high-throughput screening (HTS) methods (Bolger, Drug Discovery Today (1999) 4:251-253). Several of the assays mentioned herein can lend themselves to such screening methods. For example, cells or cell lines expressing wild type or mutant subject protein or its fragments, and a reporter gene can be subjected to compounds of interest, and depending on the reporter genes, interactions can be measured using a variety of methods such as color detection, fluorescence detection (*e.g.* GFP), autoradiography, scintillation analysis, *etc.*

Compounds identified using the above-described methods are useful to control pests, e.g., are useful as pesticides. Such compounds can control pests, e.g., by reducing pest growth, and/or fertility, and/or viability. The present invention provides compounds identified using any of the above-described assays.

### SUBJECT NUCLEIC ACIDS AS BIOPESTICIDES

Subject nucleic acids and fragments thereof, such as antisense sequences or double-stranded RNA (dsRNA), can be used to inhibit subject nucleic acid molecule function, and thus can be used as biopesticides. Methods of using dsRNA interference are described in published PCT application WO 99/32619. The biopesticides may comprise the nucleic acid molecule itself, an expression construct capable of expressing the nucleic acid, or organisms transfected with the expression construct. The biopesticides may be applied directly to plant parts or to soil surrounding the plants (e.g. to access plant parts growing beneath ground level), or directly onto the pest.

One approach well known in the art is short interfering RNA (siRNA) mediated gene silencing where expression products of an ACCase gene are targeted by specific double stranded ACCase-derived siRNA nucleotide sequences that are complementary to at least a 19-25 nt long segment (e.g., a 20-21 nucleotide sequence) of the ACCase gene transcript, including the 5' untranslated (UT) region, the ORF, or the 3' UT region. In some embodiments, short interfering RNAs are about 19-25 nt in length. See, e.g., PCT applications WO0/44895, WO99/32619, WO01/75164, WO01/92513, WO01/29058, WO01/89304, WO02/16620, and WO02/29858 for descriptions of siRNA technology.

Biopesticides comprising a subject nucleic acid may be prepared in a suitable vector for delivery to a plant or animal. For generating plants that express the subject nucleic acids, suitable vectors include *Agrobacterium tumefaciens* Ti plasmid-based vectors (Horsch *et al*., Science (1984) 233:496-89; Fraley *et al*., Proc. Natl. Acad. Sci. USA (1983) 80:4803), and recombinant cauliflower mosaic virus (Hohn *et al*., 1982, In Molecular Biology of Plant Tumors, Academic Press, New York, pp 549-560; U.S. Patent No. 4,407,956 to Howell). Retrovirus based vectors are useful for the introduction of genes into vertebrate animals (Burns *et al*., Proc. Natl. Acad. Sci. USA (1993) 90:8033-37).

Transgenic insects can be generated using a transgene comprising a subject gene operably fused to an appropriate inducible promoter. For example, a tTA-responsive promoter may be used in order to direct expression of a subject protein at an appropriate time in the life cycle of the insect. In this way, one may test efficacy as an insecticide in, for example, the larval phase of the life cycle (i.e. when feeding does the greatest damage to crops). Vectors for the introduction of genes into insects include P element (Rubin and Spradling, Science (1982) 218:348-53; U.S. Pat. No. 4,670,388), "hermes" (O'Brochta *et al*., Genetics (1996) 142:907-914), "minos" (U.S. Pat. No. 5,348,874), "mariner" (Robertson, Insect Physiol. (1995) 41:99-105), and "sleeping beauty"(Ivics *et al*., Cell (1997) 91(4):501-510), "piggyBac" (Thibault *et al*., Insect Mol Biol (1999) 8(1):119-23), and "hobo" (Atkinson *et al*., Proc. Natl. Acad. Sci. U.S.A. (1993) 90:9693-9697). Recombinant virus systems for expression of toxic proteins in infected insect cells are well known and include Semliki Forest virus (DiCiommo and Bremner, J. Biol. Chem. (1998) 273:18060-66), recombinant sindbis virus (Higgs *et al*., Insect Mol. Biol. (1995) 4:97-103; Seabaugh *et al*., Virology (1998) 243:99-112), recombinant pantropic retrovirus (Matsubara *et al*., Proc. Natl. Acad. Sci. USA (1996) 93:6181-85; Jordan *et al*., Insect Mol. Biol. (1998) 7:215-22), and recombinant baculovirus (Cory and Bishop, Mol. Biotechnol. (1997) 7(3):303-13; U.S. Patent No. 5,470,735; U.S. Patent Nos. 5,352,451; U.S. Patent No. 5, 770, 192; U.S. Patent No. 5,759,809; U.S. Patent No. 5,665,349; and U.S. Patent No. 5,554,592).

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used, e.g., bp, base pair(s); kb, kilobase(s); pl, picoliter(s); s, second(s); min, minute(s); h or hr, hour(s); aa, amino acid(s); kb, kilobase(s); bp, base pair(s); nt, nucleotide(s); and the like.

### Example 1: Cloning of a cDNA encoding Heliothis acetyl CoA carboxylase

A cDNA encoding a full-length open reading frame of an acetyl CoA carboxylase was amplified from a *Heliothis virescens* cDNA library, and sequenced in its entirety. The cDNA sequence is provided in Figures 1A-1C; the amino acid sequence of the encoded acetyl CoA carboxylase is provided in Figure 2.

Acetyl CoA Carboxylase is the rate limiting enzyme in fatty acid synthesis in most organisms. This enzyme utilizes ATP to charge a biotin functional group with a carboxyl group provided by bicarbonate; this moiety is subsequently transferred to acetyl CoA to yield malonyl CoA.

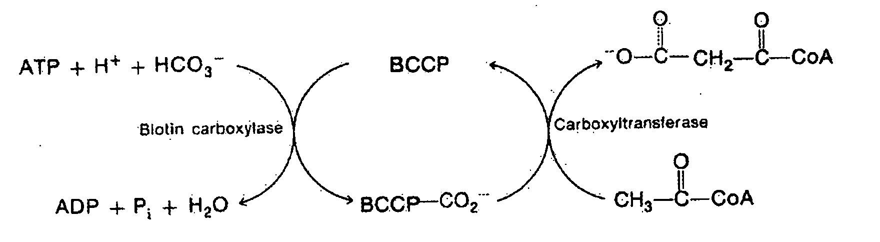

ACCase is thought to be the cognate target of B23 (nucleophosmin). Suggestive evidence was provided by the selective phenocopy of B23 treatment by RNA interference of ACCase and fatty acid synthase in *C. elegans*.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

## Claims

1. An isolated polynucleotide comprising a nucleotide sequence that encodes a polypeptide comprising the amino acid sequence set forth in SEQ ID NO:2.

2. An isolated polynucleotide comprising a nucleotide sequence having at least about 75% nucleotide sequence identity with the nucleotide sequence set forth in nucleotides 5-6859 of SEQ ID NO: 1.

3. An isolated polynucleotide comprising a nucleotide sequence that hybridizes under stringent hybridization conditions to a nucleic acid molecule having the sequence set forth in nucleotides 5-6859 of SEQ ID NO: 1.

4. A recombinant vector comprising a polynucleotide according to any one of claims 1 to 3.

5. A host cell comprising a recombinant vector according to claim 4.

6. A process for producing an insect acetyl CoA carboxylase, comprising culturing the host cell of claim 5 under conditions suitable for expression of said protein and recovering said protein.

7. A purified protein comprising an amino acid sequence having at least about 80% sequence identity with the sequence set forth in SEQ ID NO:2.

8. A method for detecting an agent that reduces an enzymatic activity of an insect acetyl CoA carboxylase, said method comprising contacting said acetyl CoA carboxylase or fragment thereof having enzymatic activity with a test agent; and determining the effect, if any, of said test agent on acetyl CoA carboxylase activity of said enzyme or fragment; wherein the amino acid sequence of said acetyl CoA carboxylase comprises an amino acid sequence amino acid sequence which is at least about 80% identical to the sequence set forth in SEQ ID NO:2.

9. The method of Claim 8, further comprising selecting a test agent that reduces acetyl CoA carboxylase activity; determining an effect, if any, of the test agent on insect viability, wherein a test agent that reduces insect viability is identified as a pesticidal agent.

10. The method of Claim 8 wherein said contacting comprises administering said test agent to cultured host cells that have been genetically engineered to produce said acetyl CoA carboxylase.

11. A method of controlling a pest, comprising contacting a pest with a compound identified by a method according to claim 8.
